# EUROPÄISCHE PATENTANMELDUNG

(11) **EP 4 261 529 A1**
(43) Veröffentlichungstag der Anmeldung: **18.10.2023**
(21) Anmeldenummer: 22168604.1
(22) Anmeldetag: 14.04.2022
(51) Int. Cl.: G01N 21/552, G01N 21/53, G01N 21/85, G01N 21/3577, G01N 21/47, G01N 33/14, G01N 33/28, G01N 21/15

(54) **AUF GESTÖRTER TOTALREFLEXION BERUHENDE SENSORANORDNUNG UND VERFAHREN ZUM BESTIMMEN EINER OPTISCHEN EIGENSCHAFT EINER TESTSUBSTANZ**

(71) Anmelder: Fraunhofer-Gesellschaft zur Förderung der angewandten Forschung e.V., 80686 München (DE)
(72) Erfinder: LAMBRECHT, Armin, 79110 Freiburg (DE); BRUNNER, Raimund, 79110 Freiburg (DE); BOLWIEN, Carsten, 79110 Freiburg (DE); SULZ, Gerd, 79110 Freiburg (DE)
(74) Vertreter: WSL Patentanwälte Partnerschaft mbB

(57) **Zusammenfassung**

Die vorliegende Erfindung betrifft eine Sensoranordnung (100) mit einem auf einer gestörten Totalreflexion beruhenden Photometer (26) zum Bestimmen einer optischen Eigenschaft einer Testsubstanz (2), wobei das Photometer (26) umfasst eine erste Strahlungsquelle (7), wobei die erste Strahlungsquelle (7) derart ausgestaltet ist, dass die erste Strahlungsquelle (7) in einem Betrieb der Sensoranordnung (100) elektromagnetische Messstrahlung erzeugt und abstrahlt, ein Reflexionselement (28), wobei das Reflexionselement (28) eine mit der Testsubstanz (2) in Kontakt bringbare Reflexionsfläche (23) aufweist, wobei das Reflexionselement (28) derart in einem Strahlengang der von der ersten Strahlungsquelle (7) abgestrahlten Messstrahlung angeordnet und derart ausgestaltet ist, dass die Messstrahlung in dem Betrieb der Sensoranordnung (100) an der Reflexionsfläche (23) eine gestörte Totalreflexion erfährt, einen ersten Strahlungsdetektor (9), wobei der erste Strahlungsdetektor (9) derart in dem Strahlengang der Messstrahlung angeordnet und derart ausgestaltet ist, dass der erste Strahlungsdetektor (9) in dem Betrieb der Sensoranordnung (100) die an der Reflexionsfläche (23) reflektierte Messstrahlung erfasst und ein die Leistung der erfassten Messstrahlung repräsentierendes Messsignal ausgibt, und eine Auswertungseinrichtung (25), wobei die Auswertungseinrichtung (25) derart mit dem ersten Strahlungsdetektor (9) verbunden ist, dass die Auswertungseinrichtung (25) in dem Betrieb der Sensoranordnung (100) das Messsignal empfängt, und wobei die Auswertungseinrichtung (25) derart eingerichtet ist, dass die Auswertungseinrichtung (25) in dem Betrieb der Sensoranordnung (100) aus dem Messsignal die optische Eigenschaft der Testsubstanz bestimmt und ein Maß für die optische Eigenschaft ausgibt, wobei die Sensoranordnung (100) einen Zustandssensor (27) aufweist, wobei der Zustandssensor (27) ein Detektorelement (8, 9, 9a, 16, 19) umfasst, wobei das Detektorelement (8, 9, 9a, 16, 19) derart angeordnet und ausgestaltet ist, dass das Detektorelement (8, 9, 9a, 16, 19) in dem Betrieb der Sensoranordnung (100) ein Maß für die Leistung einer zumindest an der Reflexionsfläche (23) gestreuten oder durch die Reflexionsfläche (23) in die Testsubstanz transmittierten und in der Testsubstanz gestreuten elektromagnetischen Streustrahlung erfasst und ein das Maß für die Leistung der Streustrahlung repräsentierendes Zustandssignal ausgibt, wobei die Auswertungseinrichtung (25) derart mit dem Detektorelement (8, 9, 9a, 16, 19) verbunden ist, dass die Auswertungseinrichtung (25) in dem Betrieb der Sensoranordnung (100) das Zustandssignal empfängt, und wobei die Auswertungseinrichtung (25) derart eingerichtet ist, dass die Auswertungseinrichtung (25) in dem Betrieb der Sensoranordnung (100) aus dem Zustandssignal einen Status zumindest der Reflexionsfläche (23) oder der Testsubstanz (2) bestimmt.

## Beschreibung

Die vorliegende Erfindung betrifft eine Sensoranordnung mit einem auf einer gestörten Totalreflexion beruhenden Photometer zum Bestimmen einer optischen Eigenschaft einer Testsubstanz, wobei das Photometer eine erste Strahlungsquelle, wobei die erste Strahlungsquelle derart ausgestaltet ist, dass die erste Strahlungsquelle in einem Betrieb der Sensoranordnung elektromagnetische Messstrahlung erzeugt und abstrahlt, ein Reflexionselement, wobei das Reflexionselement eine mit der Testsubstanz in Kontakt bringbare Reflexionsfläche aufweist, wobei das Reflexionselement derart in einem Strahlengang der von der ersten Strahlungsquelle abgestrahlten Messstrahlung angeordnet und derart ausgestaltet ist, dass die Messstrahlung in dem Betrieb der Sensoranordnung an der Reflexionsfläche eine gestörte Totalreflexion erfährt, einen ersten Strahlungsdetektor, wobei der erste Strahlungsdetektor derart unter einem ersten Winkel zu der Reflexionsfläche in dem Strahlengang der Messstrahlung angeordnet und derart ausgestaltet ist, dass der erste Strahlungsdetektor in dem Betrieb der Sensoranordnung die an der Reflexionsfläche reflektierte Messstrahlung erfasst und ein die Leistung der erfassten Messstrahlung repräsentierendes Messsignal ausgibt, und eine Auswertungseinrichtung aufweist, wobei die Auswertungseinrichtung derart mit dem ersten Strahlungsdetektor verbunden ist, dass die Auswertungseinrichtung in dem Betrieb der Sensoranordnung das Messsignal empfängt, und wobei die Auswertungseinrichtung derart eingerichtet ist, dass die Auswertungseinrichtung in dem Betrieb der Sensoranordnung aus dem Messsignal die optische Eigenschaft der Testsubstanz bestimmt und ein Maß für die optische Eigenschaft ausgibt.

Darüber hinaus betrifft die vorliegende Anmeldung ein Verfahren zum Bestimmen einer optischen Eigenschaft einer Testsubstanz mit den Schritten: in Kontakt Bringen einer Reflexionsfläche eines Reflexionselements mit der Testsubstanz, Erzeugen und Abstrahlen von elektromagnetischer Messstrahlung, Einstrahlen der Messstrahlung in das Reflexionselement, so dass die Messstrahlung an der Reflexionsfläche eine gestörte Totalreflexion erfährt, Erfassen der an der Reflexionsfläche reflektierten Messstrahlung, Erzeugen eines die Leistung der erfassten Messstrahlung repräsentierenden Messsignals, Bestimmen der optischen Eigenschaft der Testsubstanz aus dem Messsignal und Ausgeben eines Maßes für die optische Eigenschaft.

Verfahren, welche auf einer abgeschwächten oder gestörten Totalreflexion (ATR) von elektromagnetischer Strahlung beruhen, sind zur Untersuchung von Flüssigkeiten und Feststoffen in der Prozessanalytik weit verbreitet. In der Regel wird das Reflexionselement von einem Kristall oder einem Glaskörper gebildet, der für die verwendete elektromagnetische Strahlung transparent ist. Die Reflexionsfläche des Reflexionselements wird in Kontakt mit der zu analysierenden Testsubstanz gebracht. Dabei ist das Reflexionselement ein Element mit einem höheren Brechungsindex als die Testsubstanz, so dass bei Überschreiten eines Grenzwinkels die elektromagnetische Strahlung an der Grenzfläche zwischen Reflexionselement und Testsubstanz eine Totalreflexion erfährt. An der Grenzfläche bildet sich bei der Totalreflexion der elektromagnetischen Strahlung ein evaneszentes Feld aus, das sich bis in die Testsubstanz erstreckt und dessen Amplitude im Wesentlichen senkrecht zur Grenzfläche exponentiell abklingt. Dieses evaneszente Feld erfährt in der Testsubstanz in Abhängigkeit vom Absorptionsspektrum der Testsubstanz mehr oder weniger starke Absorption. Die absorbierte Leistung der Messstrahlung ist als Leistungsverlust bei einer Detektion der an der Reflexionsfläche reflektierten Messstrahlung erfassbar. Ist die Messstrahlung spektral breitbandig, so kann man, wenn man die an der Reflexionsfläche reflektierte Messstrahlung spektral aufgelöst erfasst, das Absorptionsspektrum der Testsubstanz erfassen.

Mit einem ATR-Verfahren können selbst stark absorbierende Testsubstanzen im Hinblick auf ihre Absorption untersucht werden, deren Analyse in Transmission wegen der dann notwendigerweise geringen Schichtdicken nicht untersucht werden können.

Aus dem Stand der Technik sind vielfältige Varianten und Ausführungsformen der ATR-Messtechnik bekannt. Die Verwendung von ATR-Verfahren in Online-, Atline- und Inline-Sensoren scheitert jedoch häufig an nicht ausreichender Prozesstauglichkeit der Systeme. Insbesondere führen Beschädigungen der Reflexionsfläche, Verschmutzungen und Beläge auf der Reflexionsfläche, aber auch Gasblasen und Partikelbeladungen in der Testsubstanz zu Störungen der Sensoren. Bei der Verwendung von Reflexionselementen mit einer zusätzlichen Beschichtung führt auch eine Beschädigung der Beschichtung zu solchen Störungen. Bei den ATR-Verfahren sind aber gerade aufgrund der geringen Eindringtiefe des evaneszenten Felds der Messstrahlung in die Testsubstanz sowohl Beschädigungen und/oder Verunreinigungen der Reflexionsfläche als auch Störungen in der Testsubstanz ein gravierendes Problem.

Daher ist es Aufgabe der vorliegenden Erfindung, eine Sensoranordnung und ein Verfahren, die auf einer gestörten Totalreflexion der Messstrahlung beruhen, bereitzustellen, wobei die Sensoranordnung und das Verfahren zusätzlich zu der Information über die optische Eigenschaft der Testsubstanz auch eine Zustandsinformation über einen Zustand zumindest der Sensoranordnung oder der Testsubstanz erfasst.

Zur Lösung dieser Aufgabe wird eine Sensoranordnung gemäß dem unabhängigen Anspruch 1 bereitgestellt. Dabei weist die Sensoranordnung der eingangs beschriebenen Art zusätzlich zu dem Photometer einen Zustandssensor auf. Dieser Zustandssensor umfasst ein Detektorelement, wobei das Detektorelement derart angeordnet und ausgestaltet ist, dass das Detektorelement in dem Betrieb der Sensoranordnung ein Maß für die Leistung einer zumindest an der Reflexionsfläche oder in der Testsubstanz gestreuten elektromagnetischen Streustrahlung erfasst und ein das Maß für die Leistung der Streustrahlung repräsentierendes Zustandssignal ausgibt. Die Auswertungseinrichtung ist zudem derart mit dem Detektorelement verbunden, dass die Auswertungseinrichtung in dem Betrieb der Sensoranordnung das Zustandssignal empfängt, und die Auswertungseinrichtung ist derart eingerichtet, dass die Auswertungseinrichtung in dem Betrieb der Sensoranordnung aus dem Zustandssignal einen Status der Reflexionsfläche oder der Testsubstanz bestimmt.

Es ist die grundlegende Idee der erfindungsgemäßen Sensoranordnung, dass zusätzlich zu dem Photometer ein Zustandssensor in die Sensoranordnung integriert ist. Der Zustandssensor arbeitet auf der Grundlage einer Auswertung von an der Reflexionsfläche oder in der Testsubstanz gestreuter elektromagnetischer Strahlung. Dabei ist es für den Gedanken der Integration des Photometers und des Zustandssensors in der Sensoranordnung essenziell, dass die Einstrahlung der Streuung bewirkenden elektromagnetischen Strahlung in die Reflexionsfläche oder in die Testsubstanz durch das Reflexionselement hindurch erfolgt.

Die erfindungsgemäße Lösung ermöglicht es zumindest in Ausführungsformen davon eine Reihe von Vorteilen zu realisieren: Der zusätzliche auf einer Messung eines Maßes für die Streuung beruhende Zustandssensor ermöglicht eine Einschätzung der Zuverlässigkeit, da Beeinträchtigungen der Reflexionsfläche besser erkannt werden können. Insbesondere Belegungen der Reflexionsfläche, deren Absorption spektral unauffällig sind (beispielsweise Ablagerungen aus der Testsubstanz), aber einen Austausch der Testsubstanz im evaneszenten Feld des Sensors und damit den ordnungsgemäßen Betrieb des Photometers verhindern, können so festgestellt werden. Eine defekte Reflexionsfläche kann frühzeitig erkannt und signalisiert werden und eine vorausschauende Wartung der Sensoranordnung wird möglich. Ferner ist es möglich, einen Status der Testsubstanz während der Messung mit dem Photometer zu bestimmen und zu signalisieren; Blasen, Schaumbildung, Partikel (Entmischungseffekte bei Emulsionen) können erkannt und signalisiert werden. Der Erfolg einer Reinigung kann auch ohne Testsubstanz geprüft werden (z.B. auch mit Spülmedium auf der Reflexionsfläche, bei dem die ATR-Messung keine Aussage liefert). Darüber hinaus kann die Verschmutzung der Reflexionsfläche zeitlich protokolliert werden und ggf. kann sogar der Reinigungsvorgang darüber gestartet und beendet werden. Ein kompakterer Aufbau im Vergleich zum Einsatz eines zweiten zusätzlichen Trübungssensors ist möglich.

Im Sinne der vorliegenden Erfindung umfasst ein Photometer jede Einrichtung mit einer Strahlungsquelle (im Sinne der vorliegenden Anmeldung der ersten Strahlungsquelle) zum Erzeugen der Messstrahlung, einem Reflexionselement und einem Strahlungsdetektor (im Sinne der vorliegenden Anmeldung dem ersten Strahlungsdetektor) zum Erfassen der Leistung der Messstrahlung nach einer gestörten Totalreflexion in Reflexionselement. Eine solche Leistungsänderung der Messstrahlung bei der internen Totalreflexion an der Reflexionsfläche tritt primär durch Absorption des evaneszenten Feldes in der Testsubstanz auf.

Als Strahlungsquelle für das Photometer kommen breitbandige Strahlungsquellen wie thermische Strahler und Leuchtdioden, aber auch Laser und Laserdioden in Betracht.

Das Reflexionselement ist ein für die Messstrahlung transparenter Körper mit einer vorzugsweise planen Reflexionsfläche. Beispiele für ein geeignetes Reflexionselement sind ein Kristall mit einer, vorzugsweise planen, Reflexionsfläche, wie z.B. ein Prisma oder eine rechteckige Platte mit zwei abgeschrägten Kanten zur Einkopplung zumindest der Messstrahlung in das Reflexionselement und zur Auskopplung zumindest der gestört totalreflektierten Messstrahlung aus dem Reflexionselement. In einer Ausführungsform wird auch die Zustandsstrahlung über eine der abgeschrägten Kanten in das Reflexionselement eingekoppelt. In einer weiteren Ausführungsform wird ein totalreflektierter Anteil der Zustandsstrahlung durch eine der abgeschrägten Kanten aus dem Reflexionselement ausgekoppelt.

In einer Ausführungsform der Erfindung weist das Reflexionselement auf einer der Reflexionsfläche, an welcher zumindest die Messstrahlung eine gestörte Totalreflexion erfährt, gegenüberliegenden Fläche eine reflektierende Beschichtung aus einem Metall oder zumindest einem Dielektrikum auf. Auf diese Weise wird entweder nur die Messstrahlung oder die Messstrahlung und die Zustandsstrahlung zwischen zwei Flächen hin- und herverlaufend in dem Reflexionselement geführt. Es versteht sich, dass bei einem solchen Strahlengang in einer Ausführungsform die Reflexion an der auf der Reflexionsfläche gegenüberliegenden Fläche auch auf einer internen Totalreflexion beruhen kann.

Geeignete Materialien für das Reflexionselement sind beispielsweise Saphir, Diamant, ZnSe, ZnS, CaF2, BaF2, Si, Ge, ZrO₂, MgO, GeAsTe-Glas (AMTIR) und AsSeTe-Glas (Chalkogenid-Glas).

In einer Ausführungsform der Erfindung umfasst das Reflexionselement mindestens zwei voneinander verschiedene Materialien. Dabei dient in einer Ausführungsform eine Schutzschicht, vorzugsweise eine dünne Schicht, aus einem zweiten Material dem Schutz des eigentlichen Formkörpers aus einem ersten Material. Ein Beispiel für eine solche Materialkombination ist eine Schutzschicht aus Diamant auf einem Formkörper aus Silizium. In einer solchen Ausführungsform ist die Reflexionsfläche im Sinne der vorliegenden Anmeldung die Grenzfläche zwischen der Schutzschicht des Reflexionselements und der Testsubstanz. Der Zustandssensor dient dann insbesondere auch dazu, Beschädigungen der Beschichtung zu erfassen.

In einer alternativen Ausführungsform ist auf das Reflexionselement eine Funktionsbeschichtung aufgebracht, welche im Sinne der vorliegenden Erfindung nicht zum Reflexionselement sondern zur Testsubstanz gehört. Die Reflexionsfläche des Reflexionselements ist in einer solchen Ausführungsform die Grenzfläche zwischen dem Reflexionselement und der Funktionsbeschichtung. Ein Fluid der Testsubstanz ist in Kontakt mit der Funktionsbeschichtung. In einer Ausführungsform ist die Kontaktbeschichtung porös, so dass das Fluid der Testsubstanz die Funktionsbeschichtung durchsetzt. Auf diese Weise kann sich in der Funktionsbeschichtung beispielsweise ein Bestandteil der Testsubstanz anreichern. Die Funktionsbeschichtung kann in einer Ausführungsform auch eine chemische Anbindung eines Bestandteils der Testsubstanz an die Oberfläche Funktionsbeschichtung bereitstellen. In einer Ausführungsform bewirkt ein Bestandteil des Fluids der Testsubstanz eine Veränderung oder Reaktion in der Funktionsbeschichtung. Ein Beispiel hierfür ist ein Farbumschlag der Funktionsbeschichtung bei Änderung des pH-Werts des Fluids der Testsubstanz. Auch in einer Ausführungsform mit einer Funktionsbeschichtung erfährt das sich in die Funktionsbeschichtung erstreckende evaneszente Feld der Messstrahlung eine Absorption, beispielsweise durch die Anreicherung oder den Farbumschlag, die sich mit Hilfe des Photometers erfassen lässt. Der Zustandssensor kann auch in einer solchen Ausführungsform der Erfassung eines Zustands der Funktionsbeschichtung dienen.

In einer Ausführungsform ist das Photometer so ausgestaltet, dass die von der Strahlungsquelle erzeugte Messstrahlung an der Reflexionsfläche des Reflexionselements nur eine einzige gestörte Totalreflexion erfährt. In einer alternativen Ausführungsform wird die Messstrahlung in dem Reflexionselement so geführt, dass sie eine Mehrzahl von gestörten Totalreflexionen erfährt. In einer Ausführungsform der Erfindung ist das Reflexionselement ein Wellenleiter, welcher auf einer Totalreflexion beruht, ohne dass für diese eine diskrete Anzahl von Reflexionen angegeben werden könnte.

In einer Ausführungsform der Erfindung ist der erste Strahlungsdetektor eine Thermosäule, ein Pyrometer, ein Photoleiter, z.B. HgCdTe oder PbSe, oder eine Photodiode.

In einer Ausführungsform der Erfindung arbeitet das Photometer bei einer einzigen diskreten Wellenlänge oder aber es erfasst die Leistungsänderung aufgrund der gestörten Totalreflexion der Messstrahlung integral über eine Vielzahl von Wellenlängen aus der spektralen Bandbreite der von der ersten Strahlungsquelle erzeugten und abgestrahlten Messstrahlung. In einer Ausführungsform jedoch umfasst der erste Strahlungsdetektor einen Spektrographen. Ein solcher Spektrograph ermöglicht es, die Leistung der Messstrahlung nach der gestörten Totalreflexion wellenlängenaufgelöst zu erfassen und die Eigenschaft der Testsubstanz wellenlängenabhängig zu erfassen.

Ein Beispiel für einen Spektrographen umfasst eine Anordnung aus zwei Strahlungsdetektoren mit jeweils einem Wellenlängenfilter, wobei die Strahlungsquelle die elektromagnetische Messstrahlung mit den beiden Wellenlängen erzeugt, bei denen die beiden Filter vor den Detektoren durchlässig sind.

Die Auswertungseinrichtung ist in einer Ausführungsform ein Rechner mit einer darauf ausgeführten Software.

Eine Testsubstanz im Sinne der vorliegenden Anmeldung ist eine Substanz, die bei der Wellenlänge der Messstrahlung und der Wellenlänge der Zustandsstrahlung einen geringeren Brechungsindex aufweist als das Material des Reflexionselements. Die Testsubstanz kann fest, flüssig oder gasförmig sein. Insbesondere geeignet sind die vorliegende Sensoranordnung und das Verfahren aber für flüssige Testsubstanzen. Beispiele für eine mögliche Testsubstanz umfassen insbesondere flüssige Lebensmittel, Öle usw. Insbesondere geeignet ist die Sensoranordnung zur Verwendung mit Prozessmedien, die mit der Reflexionsfläche dauerhaft oder im Durchfluss Kontakt haben. Allerdings ist es auch möglich, eine kleine Menge der Testsubstanz auf die Reflexionsfläche aufzubringen, z.B. durch Pipettieren oder Aufpressen.

In einer Ausführungsform umfasst die Sensoranordnung einen Rohrleitungsabschnitt für die Testsubstanz, wobei die Reflexionsfläche einen Abschnitt einer Wandfläche des Rohrleitungsabschnitts bildet.

In einer dazu alternativen Ausführungsform ist die Sensoranordnung als Tauchsonde ausgeführt. Eine solche Tauchsonde wird zur Bestimmung der optischen Eigenschaft mit dem Reflexionselement in einen Behälter mit der Testsubstanz eingetaucht, so dass die Reflexionsfläche in Kontakt mit der Testsubstanz ist.

Im Sinne der vorliegenden Anmeldung bezeichnet der Begriff Messstrahlung, diejenige elektromagnetische Strahlung, die von der ersten Strahlungsquelle erzeugt wird, unabhängig davon, ob diese Messstrahlung nur für die Auswertung der Absorption der Testsubstanz oder auch für die Auswertung der Streuung von elektromagnetischer Strahlung an der Reflexionsfläche oder in der Testsubstanz durch den Zustandssensor verwendet wird.

Das Messsignal gemäß der vorliegenden Anmeldung ist dasjenige Signal, welches die durch Absorption der Messstrahlung in der Testsubstanz beschriebene optische Eigenschaft der Testsubstanz repräsentiert.

Der zusätzliche Zustandssensor der Sensoranordnung dient dazu, zumindest einen Status der Reflexionsfläche und damit des Reflexionselements oder aber der Testsubstanz zu bestimmen. Die Messung des Zustandssensors beruht auf einer Messung eines Maßes für die Leistung der durch Streuung an der Reflexionsfläche oder in der Testsubstanz entstehenden Streustrahlung.

Das Zustandssignal ist dasjenige Signal, welches die Streuung entweder des evaneszenten Felds der Messstrahlung oder der Zustandsstrahlung an der Reflexionsfläche oder in der Testsubstanz oder der Messstrahlung oder der Zustandsstrahlung in der Testsubstanz beschreibt.

Das Detektorelement des Zustandssensors umfasst in einer Ausführungsform entweder einen von dem ersten Strahlungsdetektor verschiedenen zweiten Strahlungsdetektor oder wird von diesem gebildet oder das Detektorelement des Zustandssensors umfasst den ersten Strahlungsdetektor oder wird von diesem gebildet.

Eine Streuung von elektromagnetischer Strahlung an der Reflexionsfläche hat ihre Ursache in einer Veränderung der Reflexionsfläche, oder einer Veränderung einer auf der Reflexionsfläche angeordneten Funktionsbeschichtung, beispielsweise durch eine Beschädigung wie einem Kratzer oder durch die Anlagerung einer Verunreinigung auf der Reflexionsfläche. Eine Änderung der Streuung von elektromagnetischer Strahlung in der Testsubstanz hat ihre Ursache beispielsweise in einer Änderung der Konzentration von Partikeln in der Testsubstanz oder aber von Gasblasen in der Testsubstanz.

Um an der Reflexionsfläche oder in der Testsubstanz auftretende Streuung zu erfassen, gibt es eine Reihe von Alternativen sowohl für die Erzeugung der an der Reflexionsfläche oder in der Testsubstanz gestreuten elektromagnetischen Strahlung (diese wird im Sinne der vorliegenden Anmeldung als Streustrahlung bezeichnet) als auch für die Erfassung eines Maßes für die Leistung der Streustrahlung.

Damit das Messsignal, welches die optische Eigenschaft der Testsubtanz auf Grundlage einer Absorptionsmessung repräsentiert, und das Zustandssignal, welches den Zustand der Sensoranordnung oder des Testsubstand auf Grundlage einer Streulichtmessung repräsentiert voneinander unterscheidbar und daher getrennt voneinander auswertbar sind, bedarf es zumindest einer zweiten Strahlungsquelle des Zustandssensors oder eines zweiten Strahlungsdetektors des Zustandssensors. Alternativ beschrieben ist es möglich, die das Streulicht generierende elektromagnetische Strahlung mit der ersten Strahlungsquelle zu generieren und ein Maß für die Streustrahlung mit einem zweiten Strahlungsdetektor zu erfassen. Die die Streustrahlung generierende Strahlung ist dann die Messstrahlung im Sinne der vorliegenden Anmeldung, der zweite Strahlungsdetektor ist Teil des Detektorelements des Zustandssensors im Sinne der vorliegenden Anmeldung. Oder aber der Zustandssensor umfasst eine zweite Strahlungsquelle, welche Zustandsstrahlung generiert, die die Streuung erfährt. In einer solchen Variante kann das Detektorelement des Zustandssensors der erste Strahlungsdetektor des Photometers sein bzw. diesen umfassen. Selbstredend ist es möglich, dass der Zustandssensor eine von der ersten Strahlungsquelle verschiedene zweite Strahlungsquelle sowie einen von dem ersten Strahlungsdetektor verschiedenen zweiten Strahlungsdetektor aufweist.

In einer Ausführungsform der Erfindung umfasst das Detektorelement des Zustandssensors einen von dem ersten Strahlungsdetektor verschiedenen zweiten Strahlungsdetektor oder das Detektorelement ist der zweite Strahlungsdetektor, wobei der zweite Strahlungsdetektor derart angeordnet und ausgestaltet ist, dass der zweite Strahlungsdetektor in dem Betrieb der Sensoranordnung die Streustrahlung erfasst und das die Leistung der erfassten Streustrahlung repräsentierende Zustandssignal ausgibt. Mit anderen Worten ausgedrückt erfasst in einer solchen Ausführungsform der zweite Strahlungsdetektor die durch Streuung in der oben beschrieben Weise abgelenkten Photonen und nicht nur ein indirektes Maß für die Streuung durch die Bestimmung von Verlusten in der reflektierten Strahlung.

In einer Ausführungsform ist dabei der zweite Strahlungsdetektor auf der der Reflexionsfläche abgewandten Seite des Reflexionselements angeordnet. Die Streustrahlung wird in einer Ausführungsform durch das Reflexionselement transmittiert, bevor der zweite Strahlungsdetektor die Streustrahlung erfasst.

In einer alternativen Ausführungsform umfasst das Detektorelement den ersten Strahlungsdetektor oder einen zweiten Strahlungsdetektor, wobei das Detektorelement derart angeordnet und ausgestaltet ist, dass das Detektorelement in dem Betrieb der Sensoranordnung die an der Reflexionsfläche gestört totalreflektierte Messstrahlung oder eine an der Reflexionsfläche gestört totalreflektierte Zustandsstrahlung erfasst. Erfolgt eine Streuung des evaneszenten Feldes bei der gestörten Totalreflexion entweder der Messstrahlung oder der Zustandsstrahlung, so führt dies nicht nur zur "Generierung" von Streustrahlung, sondern auch zu einer Reduzierung der gestört totalreflektierten Messstrahlung oder Zustandsstrahlung. Ein Strahlungsdetektor, dies kann der erste Strahlungsdetektor oder ein gesonderter zweiter Strahlungsdetektor sein, kann daher in einer Ausführungsform der Erfindung ein Maß für die Leistung der Streustrahlung repräsentierendes Zustandssignal ausgeben.

Der Begriff Zustandsstrahlung bezeichnet elektromagnetische Strahlung, die von einer zweiten Strahlungsquelle erzeugt wurde und die ausschließlich der Erfassung des Zustands anhand der Auswertung der Streuung des evaneszenten Feldes der Zustandsstrahlung an der Reflexionsschicht oder der in die Testsubstanz transmittierten Zustandsstrahlung in der Testsubstanz verwendet wird.

In einer Ausführungsform der Erfindung ist die Streustrahlung ein gestreuter Anteil des bei der gestörten Totalreflexion an der Reflexionsfläche oder in der Testsubstanz entstehenden evaneszenten Felds der Messstrahlung oder der Zustandsstrahlung. In einer Ausführungsform der Erfindung ist die Streustrahlung ein gestreuter Anteil der durch die Reflexionsfläche in das Testmedium transmittierten Messstrahlung oder Zustandsstrahlung.

In einer Ausführungsform der Erfindung erzeugt die erste Strahlungsquelle die Messstrahlung für das Photometer (ATR-Messung). Das evaneszente Feld dieser Messstrahlung erfährt bei der Totalreflexion eine von dem Zustandssensor erfassbare Streuung an der Reflexionsfläche, sodass die Leistung der gestreuten Strahlung (im Sinne der vorliegenden Anmeldung auch als Streustrahlung bezeichnet) eine Aussage über den Status der Reflexionsfläche ermöglicht. Alternativ wird das evaneszente Feld der Streustrahlung in der Testsubstanz gestreut, und die Leistung der Streustrahlung liefert eine Information über einen Zustand der Testsubstanz, beispielsweise das Vorhandensein von Gasblasen in der Testsubstanz in unmittelbarer Nähe der Reflexionsfläche. In einer solchen Ausführungsform wird das Maß für die Leistung der Streustrahlung erfasst, indem das Detektorelement ein zweiter Strahlungsdetektor ist, wobei der zweite Strahlungsdetektor derart angeordnet ist, dass er die Streustrahlung erfasst und nicht die an der Reflexionsfläche reflektierte Messstrahlung.

In einer Ausführungsform wird zusätzlich zu der an der Reflexionsfläche reflektierten Messstrahlung ein Teil der Messstrahlung durch die Reflexionsfläche in die Testsubstanz transmittiert und in dieser gestreut werden. Wie zuvor beschrieben, wird das Maß für die Leistung der Streustrahlung erfasst, indem der zweite Strahlungsdetektor die Streustrahlung erfasst und nicht die reflektierte Messstrahlung.

In einer Ausführungsform der Erfindung ist die erste Strahlungsquelle für die Messstrahlung derart ausgestaltet, dass die Messstrahlung eine spektrale Bandbreite aufweist, wobei ein erster Wellenlängenbereich aus der spektralen Bandbreite genutzt wird, um die an der Reflexionsfläche gestört totalreflektierte Messstrahlung zu erfassen. Ein zweiter, von dem ersten Wellenlängenbereich verschiedener Wellenlängenbereich aus der spektralen Bandbreite wird genutzt, um eine Streuung des evaneszenten Feldes an der Reflexionsfläche oder in der Testsubstanz auszuwerten. Für das Erfassen des Maßes der an der Reflexionsfläche oder in der Testsubstanz gestreuten Messstrahlung hat das Detektorelement wiederum einen zweiten Strahlungsdetektor. Dieser zweite Strahlungsdetektor ist in einer Ausführungsform der Erfindung wie zuvor so angeordnet, dass er die Streustrahlung erfasst und ein die Leistung der Streustrahlung repräsentierendes Zustandssignal ausgibt.

In einer alternativen Ausführungsform der Erfindung ist der zweite Strahlungsdetektor so angeordnet, dass er wie der erste Strahlungsdetektor die an der Reflexionsfläche gestört totalreflektierte Messstrahlung erfasst. Allerdings ist der erste Strahlungsdetektor in einer solchen Ausführungsform nur selektiv sensitiv für den ersten Wellenlängenbereich und der zweite Strahlungsdetektor ist selektiv sensitiv für den zweiten Wellenlängenbereich. Der erste Strahlungsdetektor erfasst wie zuvor eine Änderung der Absorption des evaneszenten Feldes der Messstrahlung in der Testsubstanz, während der zweite Strahlungsdetektor ein Maß für die Leistung der Streustrahlung erfasst, denn die Erzeugung von Streustrahlung durch das evaneszente Feld führt auch zu einer Verringerung der an der Reflexionsfläche reflektierten Messstrahlung im zweiten Wellenlängenbereich. Eine solche Ausführungsform ist insbesondere dann sinnvoll, wenn die Testsubstanz in dem zweiten Wellenlängenbereich eine deutlich geringere Absorption aufweist als in dem ersten Wellenlängenbereich der Messstrahlung.

In einer Ausführungsform der Erfindung umfasst der Zustandssensor eine zweite Strahlungsquelle, wobei die zweite Strahlungsquelle eine elektromagnetische Strahlung erzeugt und abstrahlt, die im Sinne der vorliegenden Anmeldung als Zustandsstrahlung bezeichnet wird. Die Zustandsstrahlung weist eine von einer Wellenlänge der Messstrahlung verschiedene Wellenlänge oder eine von einer Modulationsfrequenz der Messstrahlung verschiedene Modulationsfrequenz auf.

Auch in einer solchen Ausführungsform kann die Zustandsstrahlung so in das Reflexionselement eingekoppelt werden, dass die Zustandsstrahlung eine gestörte Totalreflexion an der Reflexionsfläche erfährt. Da kann das evaneszente Feld der Zustandsstrahlung entweder an der Reflexionsfläche oder in der Testsubstanz durch Streuung die Streustrahlung generieren, die wiederum von dem zweiten Strahlungsdetektor erfasst wird. Alternativ oder zusätzlich wird die an der Reflexionsfläche reflektierte Zustandsstrahlung von dem Detektorelement erfasst.

Dabei umfasst das Detektorelement dann in einer Ausführungsform den ersten Strahlungsdetektor. In einer solchen Ausführungsform ist die erste Strahlungsquelle mit einer ersten Modulationsfrequenz und die zweite Strahlungsquelle mit einer zweiten Modulationsfrequenz moduliert, sodass sich die Leistung der Messstrahlung und die Leistung der Zustandsstrahlung mit dem ersten Strahlungsdetektor durch Filterung nach den Modulationsfrequenzen eindeutig bestimmen lassen. Die Leistung bei der Modulationsfrequenz der zweiten Strahlungsquelle ist dann ein Maß für die Leistung der durch Streuprozesse entstandenen Streustrahlung.

In einer alternativen Ausführungsform ist das Detektorelement ein zweiter Strahlungsdetektor, wobei der zweite Strahlungsdetektor derart angeordnet ist, dass der zweite Strahlungsdetektor die an der Reflexionsfläche gestört totalreflektierte Zustandsstrahlung erfasst. Die Leistung der an der Reflexionsfläche reflektierten Zustandsstrahlung ist wiederum ein Maß für die Leistung der durch Streuprozesse wie zuvor beschrieben generierten Streustrahlung.

In einer weiteren Ausführungsform der Erfindung ist die zweite Strahlungsquelle des Zustandssensors derart ausgestaltet und angeordnet, dass ein Teil der Zustandsstrahlung eine gestörte Totalreflexion an der Reflexionsfläche erfährt, während ein anderer Teil durch die Reflexionsfläche in die Testsubstanz transmittiert wird. In einer Variante einer solchen Ausführungsform der Erfindung wird die Streustrahlung mit dem Detektorelement in Form des zweiten, die Streustrahlung erfassenden Strahlungsdetektors erfasst, während ein Referenzdetektor die an der Reflexionsfläche reflektierte Zustandsstrahlung erfasst, um Leistungsschwankungen der zweiten Strahlungsquelle berücksichtigen zu können.

In einer Ausführungsform der Erfindung ist die erste Strahlungsquelle derart eingerichtet, dass die erste Strahlungsquelle in dem Betrieb der Sensoranordnung die Messstrahlung mit einer spektralen Bandbreite erzeugt und abstrahlt, wobei der erste Strahlungsdetektor derart ausgestaltet ist, dass der erste Strahlungsdetektor einen ersten Wellenlängenbereich aus der spektralen Bandbreite erfasst, wobei das Detektorelement des Zustandssensors derart ausgestaltet ist, dass das Detektorelement einen von dem ersten Wellenlängenbereich verschiedenen zweiten Wellenlängenbereich aus der spektralen Bandbreite erfasst und wobei vorzugsweise der zweite Wellenlängenbereich geringere Wellenlängen umfasst als der erste Wellenlängenbereich. Es versteht sich, dass zumindest in einer Ausführungsform der erste Wellenlängenbereich und der zweite Wellenlängenbereich keine Überschneidung aufweisen.

In einer Ausführungsform der Erfindung umfasst der Zustandssensor eine zweite Strahlungsquelle, wobei die zweite Strahlungsquelle derart ausgestaltet ist, dass die zweite Strahlungsquelle in einem Betrieb der Sensoranordnung elektromagnetische Zustandsstrahlung erzeugt und abstrahlt, wobei das Reflexionselement derart in einem Strahlengang der von der zweiten Strahlungsquelle abgestrahlten Zustandsstrahlung angeordnet und derart ausgestaltet ist, dass die Zustandsstrahlung in dem Betrieb der Sensoranordnung an der Reflexionsfläche eine gestörte Totalreflexion erfährt oder durch die Reflexionsfläche in die Testsubstanz transmittiert wird, sodass die von dem Detektorelement erfasste Streustrahlung ein an der Reflexionsfläche oder an der Testsubstanz gestreuter Anteil des bei der gestörten Totalreflexion an der Reflexionsfläche entstehenden evaneszenten Feldes der Zustandsstrahlung oder ein gestreuter Anteil der durch die Reflexionsfläche in das Testmedium transmittierten Zustandsstrahlung ist.

In einer Ausführungsform ist dabei die zweite Strahlungsquelle derart ausgestaltet, dass die zweite Strahlungsquelle die Zustandsstrahlung mit einer Zustandswellenlänge erzeugt und abstrahlt, wobei die Zustandswellenlänge von einer Messwellenlänge der Messstrahlung verschieden ist und wobei die Zustandswellenlänge vorzugsweise kleiner ist als die Messwellenlänge. Es versteht sich, dass in einer Ausführungsform der Erfindung sowohl die Messstrahlung als auch die Zustandsstrahlung jeweils eine spektrale Bandbreite aufweisen. Dabei umfasst in einer Ausführungsform die spektrale Bandbreite der Zustandsstrahlung kleinere Wellenlängen als die spektrale Bandbreite der Messstrahlung. In einer Ausführungsform der Erfindung überschneidet sich die spektrale Bandbreite der Zustandsstrahlung nicht mit der spektralen Bandbreite der Messstrahlung.

In einer Ausführungsform der Erfindung erzeugt die erste Strahlungsquelle in dem Betrieb der Sensoranordnung die Messstrahlung in einem Wellenlängenbereich von 0,8 µm bis 20 µm, d.h. im infraroten Spektralbereich. In einer Ausführungsform der Erfindung ist die erste Strahlungsquelle mit einer Frequenz in einem Bereich von 0,5 Hz bis 20 Hz moduliert.

In einer Ausführungsform der Erfindung ist die erste Strahlungsquelle ein thermischer Emitter oder eine Leuchtdiode, vorzugsweise eine Infrarotleuchtdiode. In einer solchen Ausführungsform ist die erste Strahlungsquelle vorzugsweise spektral breitbandig. In einer Ausführungsform ist die erste Strahlungsquelle ein Laser, vorzugsweise ein Infrarot-Laser wie z.B. ein Interband- oder Quantenkaskadenlaser.

In einer Ausführungsform der Erfindung erzeugt die zweite Strahlungsquelle die Zustandsstrahlung im sichtbaren Wellenlängenbereich (insbesondere in einem Wellenlängenbereich von 380 bis 750 nm) oder im nahinfraroten Spektralbereich (insbesondere in einem Wellenlängenbereich von 780 nm bis 3000 nm). In einer Ausführungsform der Erfindung ist die zweite Strahlungsquelle eine Leuchtdiode oder ein Laser.

In einer Ausführungsform der Erfindung, bei welcher das Detektorelement einen zweiten Strahlungsdetektor umfasst oder von diesem gebildet wird, wobei der zweite Strahlungsdetektor die Streustrahlung erfasst, weist der Zustandssensor zusätzlich einen Referenzdetektor auf, wobei der Referenzdetektor in einem Strahlengang eines Anteils der an der Reflexionsfläche gestört totalreflektierten reflektierten Messstrahlung mit dem zweiten Wellenlängenbereich oder der an der Reflexionsfläche gestört total reflektierten Zustandsstrahlung angeordnet und derart eingerichtet ist, dass der Referenzdetektor in dem Betrieb der Sensoranordnung eine Leistung des Anteils der Messstrahlung mit dem zweiten Wellenlängenbereich oder der Zustandsstrahlung erfasst und ein die Leistung der Messstrahlung mit dem zweiten Wellenlängenbereich oder der Zustandsstrahlung repräsentierendes Referenzsignal ausgibt, wobei die Auswertungseinrichtung derart mit dem Referenzdetektor verbunden ist, dass sie in dem Betrieb der Sensoranordnung das Referenzsignal von dem Referenzdetektor erhält, wobei die Auswertungseinrichtung derart eingerichtet ist, dass sie in dem Betrieb der Sensoranordnung das Zustandssignal mit dem Referenzsignal normiert.

Eine solche Ausführungsform ermöglicht es, Schwankungen der Leistung der Streustrahlung, welche auf eine Schwankung der Leistung bei der Erzeugung der Zustandsstrahlung oder der Messstrahlung zurückzuführen sind, herauszurechnen.

In einer Ausführungsform umfasst der zweite Strahlungsdetektor eine Photodiode, einen Photoleiter, beispielsweise PbS, PbSe, oder einen Photomultiplier.

In einer Ausführungsform, bei der der zweite Strahlungsdetektor dazu dient, die Streustrahlung zu erfassen, d.h. im Strahlengang der Streustrahlung angeordnet ist, umfasst der zweite Strahlungsdetektor ein Detektorarray, insbesondere mit einer eine zeilenförmig oder in Zeilen und Spalten angeordneten Mehrzahl von Pixeln, beispielsweise ein CCD- oder CMOS-Chip.

In einer Ausführungsform der Erfindung weist der zweite Strahlungsdetektor eine höhere Messbandbreite auf als der erste Strahlungsdetektor. Es zeigt sich, dass die Änderungen des des Status der Reflexionsfläche oder der Testsubstanz auf deutlich schnelleren Zeitskalen auftreten können als die durch die ATR-Messung erfassten Änderungen im Absorptionsvermögen der Testsubstanz. Ein Beispiel für eine solche schnelle Änderung des Status der Testsubstanz ist das Vorbeilaufen einer Gasblase an der Reflexionsfläche des Reflexionselements.

In einer Ausführungsform der Sensoranordnung ist die Auswertungseinrichtung derart eingerichtet, dass die Auswertungseinrichtung in dem Betrieb der Sensoranordnung das Messsignal und das Zustandssignal zeitlich korreliert und aus der zeitlichen Korrelation zwischen dem Messsignal und dem Zustandssignal eine Information zumindest über den Status der Reflexionsfläche und/oder des Testmediums ableitet. In einer Ausführungsform gibt der Status der Reflexionsfläche und/oder des Testmediums ein Maß für eine Vertrauenswürdigkeit des Messsignals.

Die zeitliche Korrelation des Messsignals und des Zustandssignals ermöglicht es, zusätzliche Informationen zu gewinnen, die mit einem herkömmlichen ATR-Messverfahren ohne einen integrierten Zustandssensor nicht gewonnen werden können. Mögliche auf diese Weise zu gewinnende Informationen fassen wir nachstehend bei der Behandlung des erfindungsgemäßen Verfahrens zusammen.

In einer Ausführungsform der Erfindung ist in Strahlrichtung vor dem zweiten Strahlungsdetektor eine Blende angeordnet, die ein Sichtfeld des zweiten Strahlungsdetektors begrenzt. Auf diese Weise können Mittelungseffekte, die bei der Streuung der Messstrahlung oder der Zustandsstrahlung an der Reflexionsfläche auftreten, wenn der zweite Strahlungsdetektor Streustrahlung von einer zu großen Fläche oder einem zu großen Volumen erfasst, auftreten können, minimiert werden. Zudem dient in einer Ausführungsform eine solche Blende dazu, einen Strahlungshintergrund zu reduzieren, der seine Ursache nicht in einem Streuprozess an der Reflexionsfläche oder der Testsubstanz, sondern in einer Reflexion oder Streuung an anderen Komponenten der Sensoranordnung, beispielsweise aufgrund von Justagefehlern bei Zusammenbau der Sensoranordnung, hat.

In einer weiteren Ausführungsform der Erfindung ist vor dem zweiten Strahlungsdetektor eine Linse angeordnet, wobei die Linse derart ausgestaltet ist, dass sie ein Sichtfeld des zweiten Strahlungsdetektors auf die Reflexionsfläche oder in das Testmedium fokussiert. Auch diese Maßnahme dient dazu, Mittelungseffekte und Strahlungshintergrund zu minimieren.

Soweit zuvor Aspekte der Erfindung im Hinblick auf die Sensoranordnung beschrieben wurden, so gelten diese auch für das entsprechende, nachstehend beschriebene Verfahren zum Bestimmen der optischen Eigenschaft der Testsubstanz und umgekehrt. Soweit eine Ausführungsform des Verfahrens mit einer Sensoranordnung gemäß einer Ausführungsform dieser Erfindung ausgeführt wird, so weist diese die entsprechenden Einrichtungen hierfür auf. Insbesondere sind Ausführungsformen der Vorrichtung zum Ausführen nachfolgend beschriebener Ausführungsformen des Verfahrens geeignet und eingerichtet.

Die zuvor genannte Aufgabe wird zudem auch durch ein Verfahren zum Bestimmen einer optischen Eigenschaft einer Testsubstanz gelöst, so wie es in dem auf das Verfahren gerichteten unabhängigen Anspruch definiert ist. Dabei weist das Verfahren die Schritte auf: in Kontakt Bringen einer Reflexionsfläche eines Reflexionselements mit der Testsubstanz, Erzeugen und Abstrahlen von elektromagnetischer Messstrahlung, Einstrahlen der Messstrahlung in das Reflexionselement, so dass die Messstrahlung an der Reflexionsfläche eine gestörte Totalreflexion erfährt, Erfassen der an der Reflexionsfläche reflektierten Messstrahlung, Erzeugen eines die Leistung der erfassten Messstrahlung repräsentierenden Messsignals, Bestimmen der optischen Eigenschaft der Testsubstanz aus dem Messsignal, Ausgeben eines Maßes für die optische Eigenschaft, Erfassen eines Maßes für die Leistung von zumindest an der Reflexionsfläche oder in der Testsubstanz gestreuter elektromagnetischer Streustrahlung, Erzeugen eines das Maß der Leistung der erfassten Streustrahlung repräsentierenden Zustandssignals und Bestimmen eines Status der Reflexionsfläche oder der Testsubstanz aus dem Zustandssignal.

In einer Ausführungsform des Verfahrens wird der Status der Reflexionsfläche und/oder der Testsubstanz aus dem momentanen Zustandssignal, aus einem zeitlichen Verlauf des Zustandssignals oder aus einer zeitlichen Korrelation zwischen dem zeitlichen Verlauf des Zustandssignals und einem zeitlichen Verlauf des Messsignals bestimmt. Der Status der Reflexionsfläche und/oder der Testsubstanz bestimmt in einer Ausführungsform eine Zuverlässigkeit des Messsignals und damit der Sensoranordnung.

Im Folgenden werden mögliche Effekte, die zu Korrelationen zwischen dem Messsignal und dem Zustandssignal führen können, beschrieben. Es wird erwartet, dass bei Streuereignissen an der Reflexionsfläche oder in der Testsubstanz die Leistung der reflektierten Strahlung reduziert wird und die Leistung des Streulichts dagegen zunimmt. Diese zeitliche Korrelation der beiden Signale bietet eine höhere Sicherheit bei der Detektion von Streuereignissen. Es wird erwartet, dass eine kleine Reduktion der Leistung der an der Reflexionsfläche reflektierten Strahlung schwieriger zu detektieren ist als der Anstieg eines normalerweise kleinen Maßes für die Streuung. Die zeitlichen Fluktuationen beim Durchlaufen von Gasblasen und ähnlichem entlang der Reflexionsfläche würden sich in einer Ausführungsform im Messsignal und im Zustandssignal gleichermaßen widerspiegeln. Die Frequenzspektren des Zustandssignals und des Messsignals sind in einer Ausführungsform ebenfalls korreliert. Ablagerungen in Form eines Films auf der Reflexionsfläche würden in einer Ausführungsform voraussichtlich nur eine geringe Zunahme des Zustandssignals verursachen, während eine Signalreduzierung des Messsignals erwartet wird. Bei Schaumbildung hingegen wird beispielsweise eine Zunahme des Messsignals erwartet, während gleichzeitig ein erhöhtes Zustandssignal auftritt.

Beispielsweise kann die Zuverlässigkeit der Sensoranordnung wie folgt aus dem Zustandssignal und dem Messsignal klassifiziert werden, obwohl das Messsignal des Photometers noch im spezifizierten Bereich liegt:

| **Zuverlässigkeit der Sensoranordnung** | **Photometer** | **Zustandssensor** |
|---|---|---|
| Normalbetrieb | ATR-Absorptionswerte und ATR-Intensität wie spezifiziert | Geringe Streulichtintensität (wie spezifiziert) |
| Warnung | ATR-Absorptionswerte und ATR-Intensität noch wie spezifiziert | Hohe Streulichtintensität |
| Photometer gestört | ATR-Absorptionswerte oder ATR-Intensität noch wie spezifiziert, abrupte Änderung der ATR-Intensität | Hohe Streulichtintensität, Abrupter Anstieg der Streulichtintensität |
| Testsubstanz auffällig (z.B. Gasblasen, Partikel,...) | ATR-Absorptionswerte oder ATR-Intensität noch wie spezifiziert, aber erhöhtes Rauschen | Starke Fluktuationen in den Streu lichtsignalen |

Anwendungsgebiete der vorliegenden Erfindung liegen in der Atline, Online und Inline-Prozessanalytik. Dabei werden verfahrenstechnische Produktionsprozesse analytisch begleitet, entweder durch zeitnahe Analyse eines zuvor aus dem Prozess entnommen Teils der Testsubstanz (Atline) oder durch Ausleitung eines Teils der Testsubstanz über einen Testpfad, mit welchem die Reflexionsfläche in Kontakt ist (Online), oder durch Messung direkt in einer Leitung des Prozesses (Inline). Alle drei Arten der begleitenden Prozessanalytik stellen hohe Anforderungen an die Ausfallsicherheit der verwendeten Sensoranordnung und des Verfahrens. Am höchsten ist diese Anforderung allerdings im Bereich der Inline-Prozessanalytik. Jede Wartung, Reinigung oder plötzlich auftretender Ausfall der Analytik erfordert einen Eingriff in den verfahrenstechnischen Produktionsprozess als solchen. Die Sensoranordnung kann nicht gewartet oder repariert werden, ohne dass der Produktionsprozess unterbrochen wird. In diesen Anwendungen der Prozessanalytik ist daher die erfindungsgemäße Zustandsüberwachung des Sensors besonders vorteilhaft.

Eine beispielhafte Anwendung erfolgt im Bereich der chemischen und pharmazeutischen Industrie. Beispiele für Anwendungen sind die Bestimmung einer gelösten Isocyanatkonzentration, die Bestimmung des Polymerisationsgrades von Kunststoffschmelzen und die Bestimmung der Feuchte in Kunststoffen, Lösungsmitteln oder Ölen. Beispiele für Anwendungen in der Lebensmittelindustrie sind die Bestimmung des CO₂-Gehalts, des Alkohol- oder Zuckergehalts in Getränken, die Bestimmung der Zusammensetzung von Milch oder Milchprodukten und die Zusammensetzung von Speiseölen und Fetten. Weitere Anwendungsmöglichkeiten ergeben sich in der At-Line- und In-Line-Analytik zur Wartung und Überwachung von Maschinen, beispielsweise der Bestimmung der Zusammensetzung und Alterung von Kraftstoffen, (Kühl-) Schmierstoffen, Getriebeöl, Kompressoröl, Transformatorenöl, Reinigungsflüssigkeiten, usw. Auch im Bereich der Biotechnologie kann die beschriebene Sensoranordnung zum Einsatz kommen, beispielsweise zur Überwachung von Bioreaktoren.

Weitere Vorteile, Merkmale und Anwendungsmöglichkeiten der vorliegenden Erfindung werden anhand der folgenden Beschreibung von Ausführungsformen und den dazugehörigen Figuren deutlich. In den Figuren sind gleiche Elemente mit gleichen Bezugszeichen bezeichnet.
- Figur 1: ist eine schematische Darstellung einer ersten Ausführungsform der erfindungsgemäßen Sensoranordnung.
- Figur 2: ist eine schematische Darstellung einer zweiten Ausführungsform der erfindungsgemäßen Sensoranordnung.
- Figur 3: ist eine schematische Darstellung einer dritten Ausführungsform der erfindungsgemäßen Sensoranordnung.
- Figur 4: ist eine schematische Darstellung einer vierten Ausführungsform der erfindungsgemäßen Sensoranordnung.
- Figur 5: ist eine schematische Darstellung eines Teils einer fünften Ausführungsform der erfindungsgemäßen Sensoranordnung mit einer flächigen Erfassung des Streulichts.
- Figur 6: ist eine schematische Darstellung einer fasergekoppelten sechsten Ausführungsform der erfindungsgemäßen Sensoranordnung.
- Figur 7: ist eine schematische Darstellung einer alternativen Geometrie des Reflexionselements der Sensoranordnung.
- Figur 8: ist eine schematische Darstellung einer weiteren möglichen Geometrie des Reflexionselements der Sensoranordnung.
Im Hinblick auf das Photometer 26 ist das Messprinzip für alle Ausführungsformen das gleiche, auch wenn die Ausgestaltungen der ersten Strahlungsquelle 3, welche die Messstrahlung 3 generiert, des Reflexionselements 28 und des ersten Strahlungsdetektors 9, welcher die Messstrahlung hinter dem Reflexionselement 28erfasst, unterschiedlich sind. Die Anordnung aus der ersten Strahlungsquelle 7, dem Reflexionselement 28, dem ersten Strahlungsdetektor 9 und der schematisch angedeuteten Auswertungseinrichtung 25 bildet das Photometer 26 im Sinne der vorliegenden Anmeldung. In den dargestellten Ausführungsformen ist die Testsubstanz 2 ein flüssiges Lebensmittel, beispielsweise Milch.

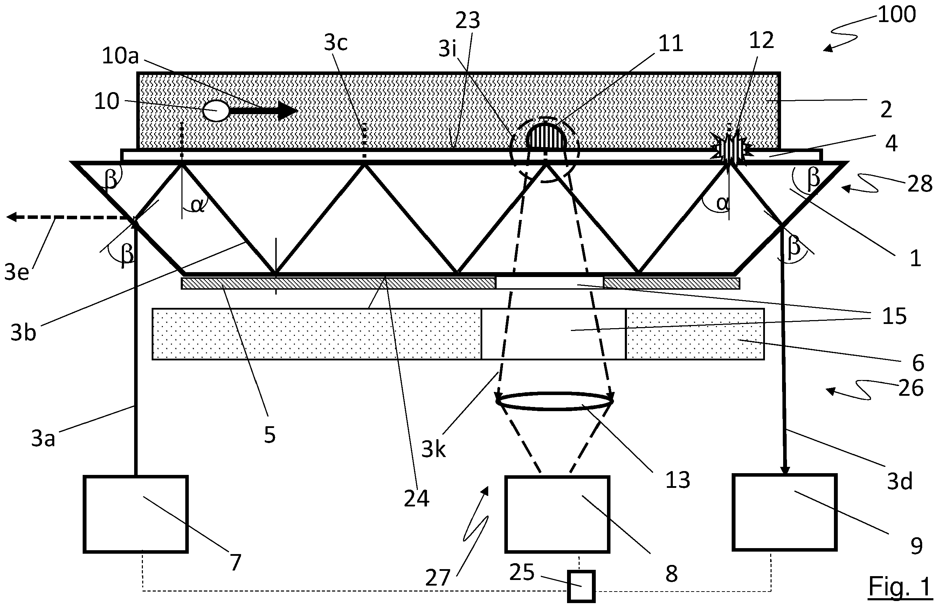

Alle gezeigten Ausführungsformen der Sensoranordnung 100 beruhen auf einer Absorptionsmessung einer Testsubstanz 2 mithilfe einer gestörten Totalreflexion an einer Reflexionsfläche 23 eines Reflexionselements 28. In den dargestellten Ausführungsformen der Figuren 1 bis 3 umfasst das Reflexionselement 28 einen Siliziumkristall 1, welcher für infrarote Strahlung transparent ist. Soweit erforderlich umfasst das Reflexionselement 28 eine zur Testsubstanz 2 hin auf den Kristall 1 aufgebrachte Schutzschicht 4. Die Schutzschicht 4 ist ebenfalls für infrarote Strahlung transparent und dient dazu, einen Schutz des Kristalls 1 bereitzustellen. Auf der der Reflexionsfläche 23 gegenüberliegenden Fläche 24 ist das Reflexionselement 28 mit einer reflektierenden Metallisierung 5, im vorliegenden Beispiel Gold, versehen. In Ausführungsformen dient die Goldbeschichtung 5 auch zur Ausbildung einer Blende für die noch zu beschreibende Streulichtmessung. Das Reflexionselement 28 ist auf einer Trägerplatte 6 aus Kunststoff montiert, wobei die Trägerplatte 6 in den Ausführungsformen, in denen dies erforderlich ist, eine Öffnung 15 zum Durchleiten des Streulichts aufweist.

Alle Ausführungsformen dienen dazu, eine optische Eigenschaft der Testsubstanz 2 mit Hilfe von Messstrahlung 3 zu erfassen. In den Figuren ist die Messstrahlung mit der Ziffer 3 als Bezugszeichen bezeichnet, wobei der Messstrahlung von der ersten Strahlungsquelle 7 unterschiedliche kleine Buchstaben nachgestellt sind, welche dazu dienen, die Messstrahlung an unterschiedlichen Orten im Strahlengang zu bezeichnen.

Dabei wird davon ausgegangen, dass die Testsubstanz 2 im Infraroten eine sehr hohe Absorption aufweist. Daher arbeitet das Photometer 26 in allen Ausführungsformen mit einer gestörten Totalreflexion der Messstrahlung 3b an der Reflexionsfläche 23 des Reflexionselements 28. Ein evaneszentes Feld 3c der Strahlung tritt bei der internen Totalreflexion an der Reflexionsfläche 23 in die Testsubstanz 2 ein und wird dort wellenlängenabhängig zumindest teilweise absorbiert. Diese Absorption des evaneszenten Feldes 3c in der Testsubstanz 2 führt zu einer Störung der internen Totalreflexion an der Reflexionsfläche 23. Die durch diese Störung der Totalreflexion bewirkte Schwächung der Leistung der in dem Reflexionselement 28 zwischen der Reflexionsfläche 23 und der Metallisierung 5 hin und her reflektierten Strahlung wird nach Austritt aus dem Reflexionselement mit einem Strahlungsdetektor 9 gemessen. Der Strahlungsdetektor 9 bildet im Sinne der vorliegenden Anmeldung den ersten Strahlungsdetektor der Sensoranordnung 100 und damit den ATR-Detektor des Photometers 26.

In Varianten der dargestellten Ausführungsformen erfolgt die Erfassung der Messstrahlung 3d in Strahlrichtung hinter dem Reflexionselement 28 mit dem ATR-Detektor 9 spektral aufgelöst, d.h. der ATR-Detektor 9 ist dann ein Spektrograph.

In der Ausführungsform aus Figur 1 ist die erste Strahlungsquelle 7 zum Erzeugen der Messstrahlung 3 ein breitbandiger inkohärenter thermischer Emitter, welcher einen breiten Spektralbereich in einem Bereich von 0,85 µm bis 20 µm Wellenlänge abdeckt. Der erste Strahlungsdetektor 9 ist in der Ausführungsform aus Figur 1 ein Thermopile mit einem davor angeordneten Spektralfilter, wobei das Spektralfilter so gewählt ist, dass es zu der Testsubstanz 2 passt.

Befindet sich in der Testsubstanz 2 im Bereich des evaneszenten Feldes 3c eine Gasblase 10, ein Oberflächenbelag 11, welcher an der Beschichtung 4 der Reflexionsfläche 23 anhaftet oder aber ein Oberflächendefekt 12, z.B. ein Kratzer, in der Reflexionsfläche 23 des Reflexionselements 1, so wird das evaneszente Feld 3c an diesen gestreut. Die verschiedenen Ursachen für Streuprozesse sind in den Figuren schematisch dargestellt. Diese Streuung wird mit dem Zustandssensor 27 der Sensoranordnung 100 zusätzlich zu der Störung der Totalreflexion aufgrund der Testsubstanz 2 erfasst.

Der von der Reflexionsfläche 23 oder der Testsubstanz 2 gestreute Anteil an elektromagnetischer Strahlung wird als Streustrahlung 3k im Sinne der vorliegenden Anmeldung bezeichnet und bildet ein quantitatives Maß für einen Status der Reflexionsfläche 23 bzw. der Testsubstanz 2 und damit für die Sensoranordnung bzw. für das implementierte Messverfahren.

Zur Überwachung dieses Zustands mittels der generierten Streustrahlung 3k gibt es eine Reihe von Varianten, die nun anhand der Figuren skizziert werden.

In Figur 1 wird ein Teil 3k des Streulichts 3i durch den ATR-Kristall 1 transmittiert und über eine Optik 13 auf den Streulichtdetektor 8 (zweiter Strahlungsdetektor im Sinne der vorliegenden Anmeldung) geleitet. Zur Unterdrückung von Hintergrundstrahlung oder Streulicht, das nicht durch das evaneszente Feld 3c angeregt ist, dient auch die Metallisierung 5, die im Bereich des Sichtfeldes des Streulichtdetektors 8 ausgespart ist, sodass die Metallisierung 5 eine Blende bildet. Es versteht sich, dass mit der Anordnung aus Figur 1 eine Streuung durch einen Störprozess nur dann erfasst werden kann, wenn die Streuung, anders als beispielsweise für die Gasblase 10 aus Figur 1 gezeigt, im Blickfeld des zweiten Strahlungsdetektors 8 angeordnet ist.

Der zweite Strahlungsdetektor 8 als Teil des Zustandssensors wird auch Streulichtdetektor bezeichnet. Der Streulichtdetektor 8 erfasst das Streulicht 3k mit hoher Empfindlichkeit und eine großen Detektionsbandbreite, um auch sehr schwache Streulichtanteile 3k zu erfassen. Insbesondere kann bei der Verwendung einer breitbandigen thermischen ersten Strahlungsquelle 7 dabei der Anteil der elektromagnetischen Strahlung 3 im Nahinfraroten für die Zustandsmessung genutzt werden, da dieser von der Testsubstanz 2 nicht so stark absorbiert wird, wie die längerwelligen Anteile der Strahlung.

In der dargestellten Ausführungsform hat der Streulichtdetektor eine deutlich höhere Detektionsbandbreite als der ATR-Detektor 9 und kann daher schnelle Veränderungen im Millisekundenbereich erfassen. So können zuverlässig sowohl langsame Veränderungen der Reflexionsfläche 23 bzw. der Beschichtung 4 aber auch dynamische Prozesse wie das Passieren von Gasblasen 10 erkannt werden.

Die in Figur 1 gezeigte fokussierte punktuelle Erfassung der Streustrahlung entlang einer Stelle der Reflexionsfläche 23 ist zur Beobachtung von dynamischen Prozessen besser geeignet als eine flächige Detektion, da sich gleichzeitig an unterschiedlichen Orten auf der Reflexionsfläche 23 auftretende Prozesse nicht herausmitteln.

In der in Figur 1 dargestellten Ausführungsform beruhen alle Streulichteffekte, die von dem Streulichtdetektor 8 erfasst werden, auf einer Streuung des evaneszenten Feldes 3c der Messstrahlung 3.

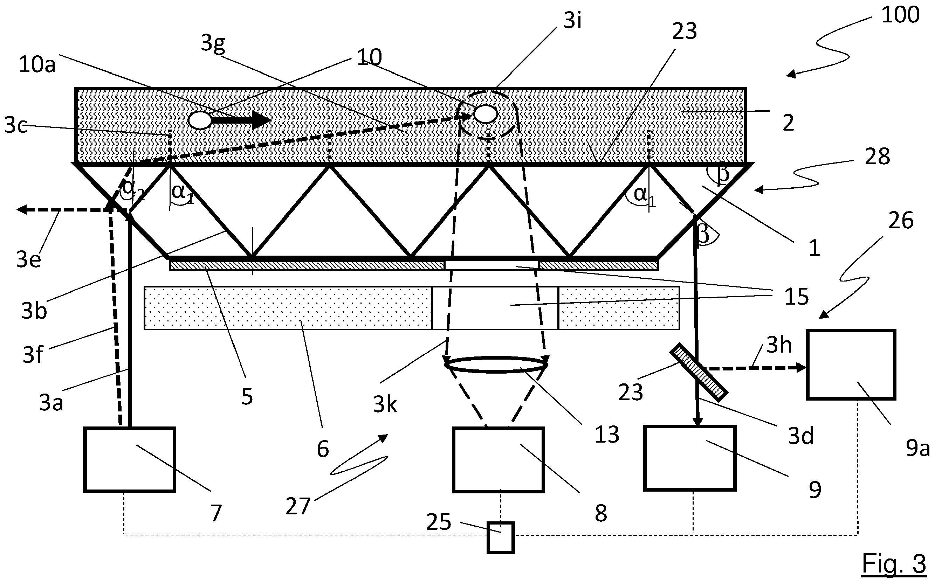

Im Gegensatz dazu zeigt die Ausführungsform aus Figur 3 eine Variante, bei der ausgenutzt wird, dass die inkohärente Strahlungsquelle 7 nur begrenzt kollimierbar ist und die Strahlung 3a, 3f daher divergent ist. Der Grad der Divergenz kann durch Vorsatzoptiken oder Blenden in Grenzen eingestellt werden. Divergente Strahlungsanteile der Messstrahlung 3a, 3f vor dem Kristall 1 werden unter unterschiedlichen Winkeln in den Kristall 1 eingekoppelt. In dem in Figur 3 gezeigten Fall unterschreitet der mit 3f bezeichnete Anteil der divergenten Messstrahlung aus der ersten Strahlungsquelle 7 daher den Grenzwinkel für die Totalreflexion und der Strahlungsanteil 3f wird durch die Reflexionsfläche 23 in die Testsubstanz 2 transmittiert. Dort ist die transmittierte Messstrahlung mit dem Bezugszeichen 3g bezeichnet. Mit dieser Art der Beleuchtung werden auch Gasblasen 10 oder Schmutzpartikel in der Testsubstanz, die sich weiter von der Reflexionsfläche 23 entfernt befinden, zur Streuung angeregt. Voraussetzung für eine hinreichend hohe Eindringtiefe der transmittierten Messstrahlung 3g in die Testsubstanz 2 ist aber eine ausreichend geringe Absorption der für die Streumessung mit dem Streulichtdetektor 8 genutzten spektralen Anteile der Messstrahlung 3.

Grundsätzlich ist für den ATR-Sensor (Photometer 26) die Wahl des Einfallswinkels α1 nahe am Grenzwinkel der internen Totalreflexion vorteilhaft, da so die höchste Empfindlichkeit für die ATR-Messung erreicht wird. Andererseits sollte der in die Testsubstanz transmittierte Strahlungsanteil 3g bezogen auf seine Leistung gering sein, da anderenfalls die für den ATR-Effekt notwendige Strahlungsleistung der Strahlung 3b reduziert ist.

Das Streulicht 3k enthält in der Ausführungsform aus Figur 3 sowohl einen Anteil aus der Streuung des evaneszenten Feldes 3c als auch aus der Streuung der transmittierten Strahlung 3g in der Testsubstanz 2. Die jeweiligen Anteile lassen sich durch Wahl des Einfallswinkels α1, Wahl und Orientierung der ersten Strahlungsquelle 7 und von eventuellen Kollimationsoptiken oder Blenden bestimmen und an die adressierte Anwendung anpassen.

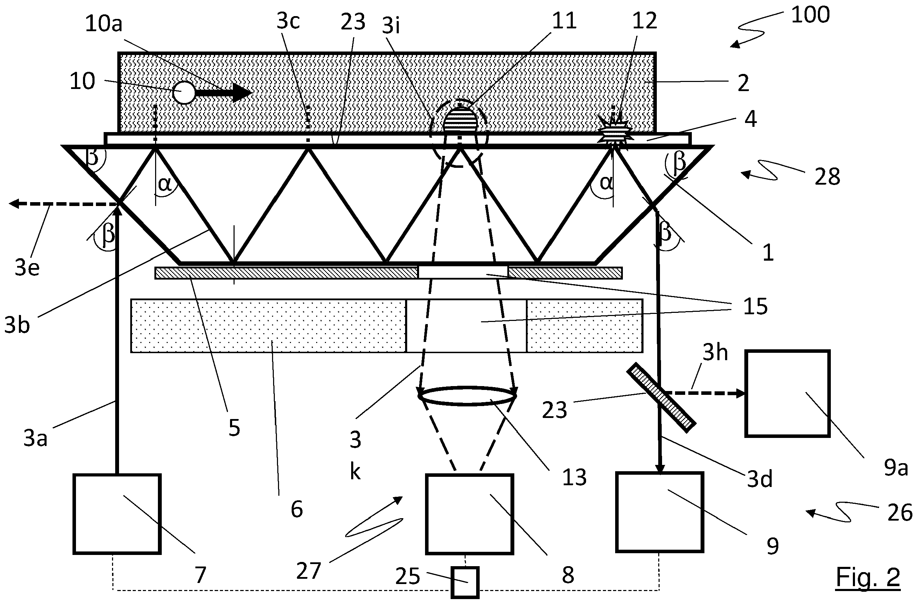

In der Ausführungsform aus Figur 1 beruht die Generation von Streulicht ausschließlich auf Streuprozessen, aufgrund des evaneszenten Feldes 3c der Messstrahlung. Daher wird in der Ausführungsform aus Figur 2 zusätzlich zu dem Streulicht 3k der kürzerwellige Anteil auch der gestört an der Reflexionsfläche 23 totalreflektierten Messstrahlung hin in Strahlrichtung hinter dem Kristall 1 erfasst. Dazu ist hinter dem ATR-Kristall 1 ein dichroitischer Spiegel 23 angeordnet, der nur dem Anteil 3h der Messstrahlung mit kürzerer Wellenlänge auf einen Referenzdetektor 9a leitet. Änderungen in der Intensität des Streulichts 3k in dem zweiten Strahlungsdetektor 8 können dann herausgerechnet werden, wenn sie beispielsweise auf eine Schwankung der Leistung der ersten Strahlungsquelle 7 zurückzuführen sind. Solche Leistungsschwankungen sind auch auf dem Signal des Referenzdetektors 9a zu sehen. Der Referenzdetektor 9a ist Bestandteil des Zustandssensors 27, da er dazu dient, die Streuprozesse zu erfassen, welche den Zustand der Sensoranordnung 100 beschreiben.

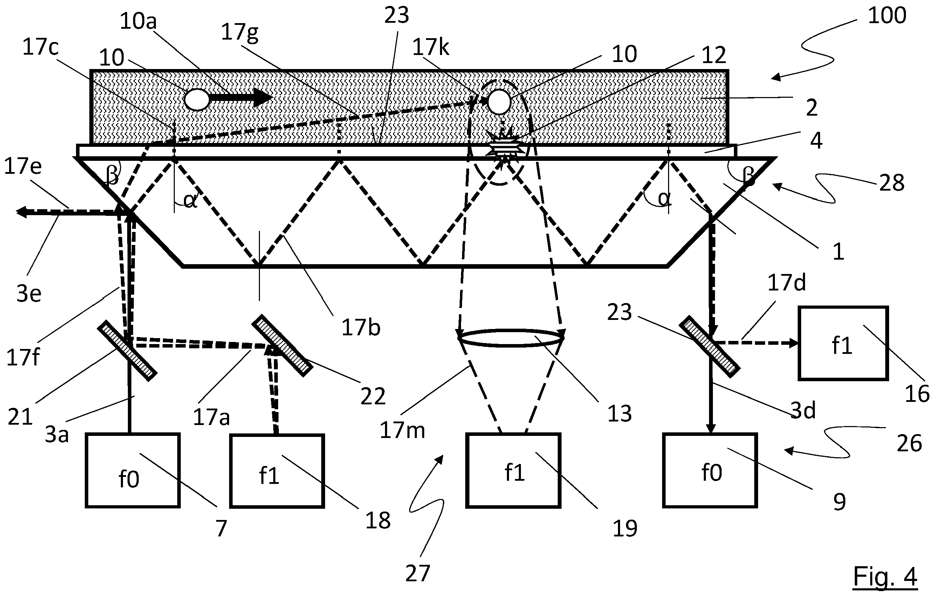

Figur 4 zeigt eine weitere alternative Ausführungsform der Sensoranordnung 100. Dabei unterscheidet sich die Sensoranordnung 100 gemäß Figur 4 von den zuvor diskutierten Ausführungsformen insbesondere dadurch, dass sie neben der ersten Strahlungsquelle 7 für die Messstrahlung 3 eine zweite Strahlungsquelle 18 aufweist. Diese zweite Strahlungsquelle 18 ist nur dem Zustandssensor 27 zugeordnet, d.h. sie erzeugt elektromagnetische Strahlung, die im Sinne der vorliegenden Anmeldung als Zustandsstrahlung bezeichnet ist und in Figur 4 mit der Ziffer 17 und je nach Ort der Betrachtung dieser Zustandsstrahlung mit einem kleinen Buchstaben a bis d bezeichnet ist. Die Zustandsstrahlung 17 ist diejenige Strahlung, die von der zweiten Strahlungsquelle 18 erzeugt wird, um den Zustand der Testsubstanz 2 oder der Reflexionsfläche 23 anhand von Streuprozessen zu erfassen.

Die zweite Strahlungsquelle 18 erzeugt in der dargestellten Ausführungsform eine Zustandsstrahlung 17 im sichtbaren Spektralbereich. Die zweite Strahlungsquelle 18 ist ein Halbleiterlaser und dieser wird entweder so in das Reflexionselement 28 eingekoppelt, dass die Zustandsstrahlung wie auch die Messstrahlung 3 eine gestörte Totalreflexion an der Reflexionsfläche 23 erfährt und/oder die Zustandsstrahlung 17 wird so in das Reflexionselement 28 eingekoppelt, dass die Zustandsstrahlung durch die Reflexionsfläche 23 in die Testsubstanz 2 transmittiert wird. Das Reflexionselement 28 muss sowohl für die Messstrahlung 3 als auch für die Zustandsstrahlung 17 transparent sein. Daher ist in der dargestellten Ausführungsform das Reflexionselement 1 ein aus Zinkselenid (ZnSe) hergestellter ATR-Kristall.

Der wesentliche Vorteil der Ausführungsform aus Figur 4 liegt in der höheren Streulichtempfindlichkeit durch die höhere Intensität der Zustandsstrahlung gegenüber dem Strahlungsanteil der Messstrahlung, deren Streuprozesse in den Figuren 1 bis 3 erfasst werden. Die zweite Strahlungsquelle 18 erlaubt es zudem, eine deutlich andere Wellenlänge für die Zustandsstrahlung zu verwenden und darüber hinaus durch unterschiedliche Modulationsfrequenzen f0 für die erste Strahlungsquelle und f1 für die zweite Strahlungsquelle ein Übersprechen zwischen dem Messsignal und dem Zustandssignal zu verhindern. Entsprechend erfolgt die Detektion des Messsignals mittels eines Lock-in-Verstärkers bei der Frequenz f0 und des Zustandssignals bei der Frequenz f1.

Wie zuvor beschrieben, können unterschiedliche Störungen entweder der Reflexionsfläche oder der Testsubstanz 2 erfasst werden. In einer solchen Ausführungsform gibt es aber zwei verschiedene Möglichkeiten, ein Maß für die entweder von der Reflexionsfläche 23 oder die in der Testsubstanz 2 gestreuten Zustandsstrahlung zu erfassen.

In einer ersten Variante sieht die Anordnung einen zweiten Strahlungsdetektor, in Figur 4 mit 19 bezeichnet, vor, der die gleiche Funktionalität bereitstellt, wie der Strahlungsdetektor 8 aus den Figuren 1 bis 3. Wie zuvor beschrieben, kann zusätzlich zu dem zweiten Strahlungsdetektor 19 der Strahlungsdetektor 16 zusammen mit dem dichroitischen Spiegel 23 als Referenzdetektor für die an der Reflexionsfläche 23 reflektierte Zustandsstrahlung 17d verwendet werden.

Allerdings ist es in der in Figur 4 gezeigten Variante auch möglich, den zweiten Strahlungsdetektor 19 durch den Detektor 16 zu ersetzen. Dieser bildet dann das Detektionselement des Zustandssensors im Sinne der vorliegenden Anmeldung. Auch Streuprozesse, welche durch die Zustandsstrahlung 17 angeregt werden, führen dann, wenn sie vom evaneszenten Feld 17c der Zustandsstrahlung bei der gestörten Totalreflexion an der Reflexionsfläche 23 herrühren, zu einem Leistungsverlust in der Zustandsstrahlung 17d, welche den ATR-Kristall 1 verlässt. Mit dem zweiten Strahlungsdetektor 16 wird dann allerdings nicht die Leistung der Streulichtstrahlung 17m erfasst, sondern nur ein Maß für die gestreute Leistung (dieses fehlt in der reflektierten Leistung).

In der Ausführungsform aus Figur 4 kann die Kombination aus dem zweiten Strahlungsdetektor 19 und dem Strahlungsdetektor 16 genutzt werden, um sowohl Streuprozesse, die in der Testsubstanz 2 auftreten als auch Streuprozesse, die im evaneszenten Feld der Zustandsstrahlung 17, die eine interne Totalreflexion an der Reflexionsfläche 23 erfährt, zu quantifizieren. Während der Strahlungsdetektor 19 Streulicht von beiden Prozessen einfängt, sieht der Detektor 16 nur Streulichtprozesse, die auf eine Streuung des evaneszenten Feldes 17c zurückgehen.

Grundsätzlich wäre es sogar möglich, wenn man für den ersten Strahlungsdetektor 9 eine hinreichend schnelle und sowohl für den Wellenlängenbereich der Messstrahlung 3 als auch für den Wellenlängenbereich der Zustandsstrahlung 17 hinreichend sensitive Photodiode verwendet, sowohl das Messsignals als auch das Zustandssignal mithilfe des Strahlungsdetektors 9 zu erfassen. Dieser Strahlungsdetektor 9 ist dann der erste Strahlungsdetektor im Sinne der vorliegenden Anmeldung und gleichzeitig auch das Detektorelement des Zustandssensors. Da die beiden Strahlungsquellen 7, 18 mit unterschiedlichen Modulationsfrequenzen moduliert sind, lassen sich mittels Lock-in-Technik die beiden Signale aus dem Ausgang des Strahlungsdetektors 9 ableiten. In diesem Falle hätte der Aufbau aus Figur 4 nur den Strahlungsdetektor 9, nicht aber die Detektoren 19 und 16.

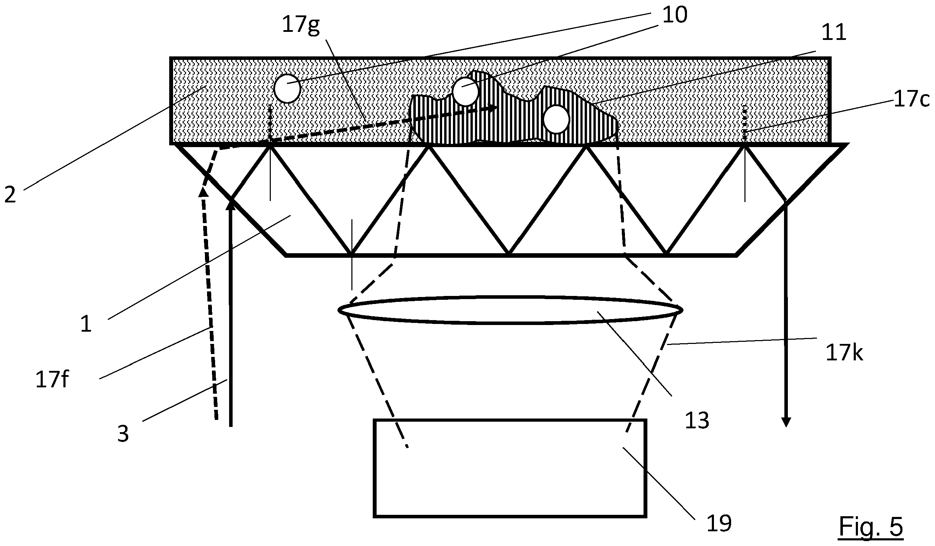

Figur 5 zeigt eine Variante der Ausführungsform aus Figur 4. Statt des die Reflexionsfläche 23 und die Testsubstanz 2 punktförmig beobachtenden Strahlungsdetektors 19 kommt ein flächiger, ortsauflösender CCD-Chip 19 mit einer davon angeordneten Optik 13 als zweiter Strahlungsdetektor zur Erfassung des Streulichts 17k zum Einsatz. Die Beleuchtung für die ATR-Messung und die Streulichtanregung erfolgt in gleicher Weise wie zuvor für Figur 4 beschrieben. Mittels der flächigen Detektion sind zum einen die Streulichtsignale aller Totalreflexionen im Reflexionselement 28, bei denen ein evaneszentes Feld auftritt, erfassbar. Zum anderen sind über die gesamte Oberfläche des Reflexionselements 28 hinweg Streusignale aus der Testsubstanz 2 oberhalb des Reflexionselements 28 erfassbar.

Die Dynamik einzelner Streuprozesse kann dabei zwar nicht so gut erfasst werden, wie in der Ausführungsform gemäß Figur 4, da sich mehrere Streuereignisse entlang der Erstreckung des Reflexionselements 28 zeitlich überlagern. Dafür kann so ein Mittelwert über die Beschaffenheit der Reflexionsfläche 23 bzw. deren Veränderung bestimmt werden, der dann als Zustandssignal ausgegeben und angezeigt wird. Ein solches Zustandssignal kann beispielsweise als Indiz für eine anstehende Wartung verwendet werden.

Es versteht sich, dass eine flächige Erfassung, so wie sie beispielhaft in Figur 5 für die Variante aus Figur 4 beschrieben ist, auch möglich ist für die Ausführungsformen der Figuren 1 bis 3, welche nur mit einer ersten Strahlungsquelle für die Messstrahlung 3 auskommen.

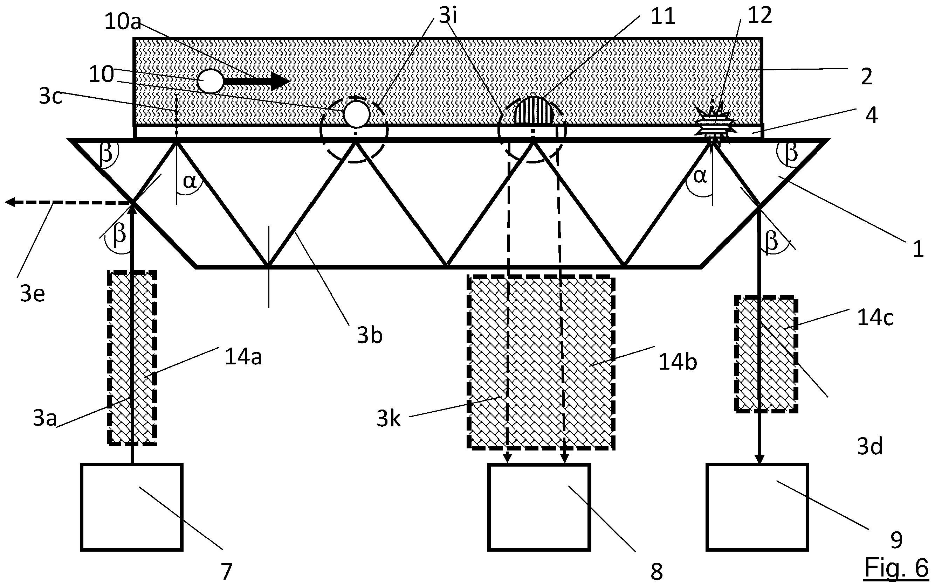

Figur 6 zeigt schematisch auf Grundlage der Ausführungsform aus Figur 1, dass es grundsätzlich möglich ist, sowohl Strahlengänge, welche die Messstrahlung 3 in das Reflexionselement 28 einkoppeln, als auch solche, die die Strahlung aus dem Reflexionselement 28 auskoppeln, fasergeführt zu implementieren. Dazu sind in der Ausführungsform aus Figur 6 drei Faserabschnitte 14a, 14b und 14c schematisch angedeutet. Vorteile einer solchen Faserkopplung sind eine schlankere Bauform, beispielweise zur Realisierung einer Tauchsonde, und eine Eignung zur Messung bei höheren Prozesstemperaturen, da Strahlungsquelle(n), Strahlungsdetektor(en) und Auswertungseinrichtung nicht den hohen Prozesstemperaturen ausgesetzt sein müssen.

In den bisher anhand der Ausführungsformen der Figuren 1 bis 6 diskutierten Varianten wurde die Einkopplung der Messstrahlung 3 und der Zustandsstrahlung 17 in den ATR-Kristall 1 von unten bei einem Winkel β von etwa 40° demonstriert. Diese Form der Einkopplung ist für hochbrechende ATR-Kristalle, z.B. aus Silizium oder Germanium, optimal. Für diese Materialien ist der Grenzwinkel der Totalreflexion z.B. für wässrige Prozessmedien kleiner als 30°.

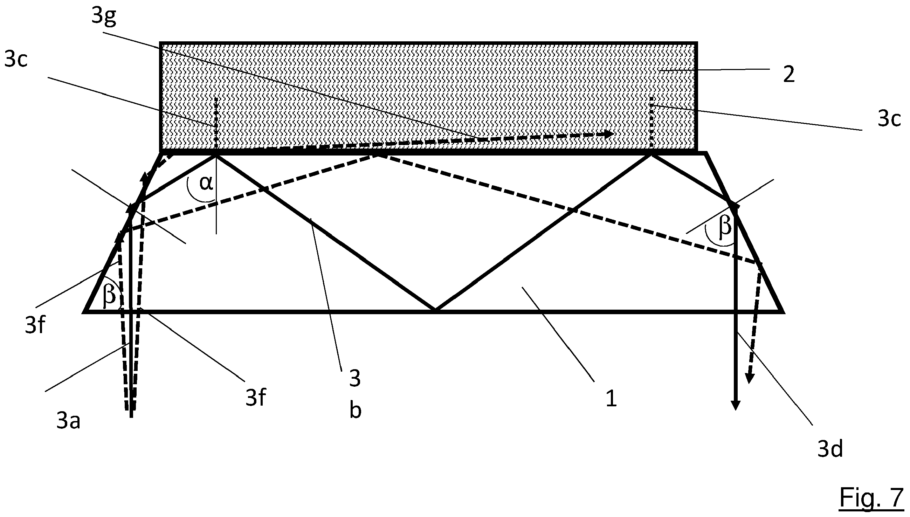

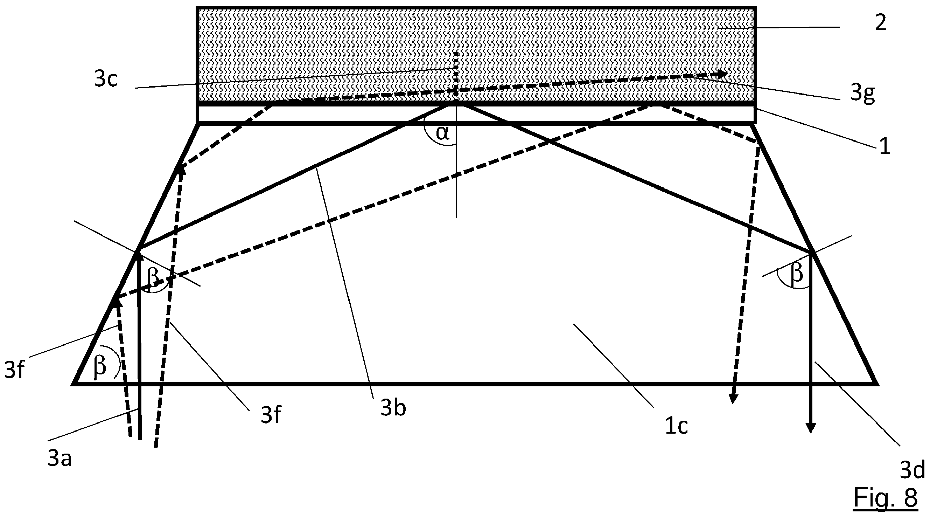

Für ATR-Kristalle 1 aus Materialien mit niedrigerem Brechungsindex wie z.B. Saphir, ZnSe, Diamant, CaF2, usw. sind andere Anordnung mitunter besser geeignet, so wie es die Figuren 7 und 8 zeigen. Die übrigen Merkmale der oben beschriebenen Ausführungsformen lassen sich auf die Einkoppelgeometrien aus den Figuren 7 und 8 ohne Weiteres übertragen.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschrieben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

### Bezugszeichenliste

- 1: ATR-Kristall des Reflexionselements 28
- 2: Testsubstanz (Prozessflüssigkeit z.B. Getränk, Öl)
- 3: Messstrahlung für die ATR-Messung
- 3a: Messstrahlung vor Eintritt in das Reflexionselement
- 3b: totalreflektierte Messstrahlung in das Reflexionselement
- 3c: Evaneszentes Feld der Messstrahlung
- 3d: Messtrahlung nach Austritt aus dem Reflexionselement vor Eintritt in den ersten Strahlungsdetektor
- 3e: an der Eintrittsstelle in das Reflexionselement reflektierte Messstrahlung
- 3f: Divergente Messstrahlung
- 3g: in die Testsubstanz transmittierte Messstrahlung
- 3h: Kurzwelliger Strahlungsanteil der Messstrahlung
- 3i: Durch Gasblasen, Partikel oder Oberflächendefekte gestreute Messstrahlung
- 3k: Streustrahlung
- 4: Schutzschicht des Reflexionselements 28
- 5: Metallisierung
- 6: Trägerplatte
- 7: erste Strahlungsquelle
- 8: zweiter Strahlungsdetektor zum Erfassen der Streustrahlung 3k
- 9: erster Strahlungsdetektor (ATR-Detektor) zum Erfassen der reflektierten Strahlung 3d
- 9a: Referenzdetektor
- 10: Gasblase
- 10a: Strömungsrichtung der Testsubstanz
- 11: partikuläre Verschmutzung an der Reflexionsfläche
- 12: Oberflächendefekt in der Beschichtung 4 oder der Reflexionsfläche 23
- 13: Abbildungsoptik für Streulicht
- 14a: Lichtleiter (z.B. Lightpipes oder Fasern) für erste Strahlungsquelle
- 14b: Lichtleiter (z.B. Lightpipes oder Fasern) für Streulichtdetektion
- 14c: Lichtleiter (z.B. Lightpipes oder Fasern) für ATR-Detektion
- 15: Öffnung in Metallisierung und Trägerplatte
- 16: zweiter Strahlungsdetektor oder Referenzdetektor
- 17: Zustandsstrahlung
- 17a: Beleuchtungsstrahl der Zustandsstrahlung
- 17b: totalreflektierte Zustandsstrahlung
- 17c: evaneszentes Feld der Zustandsstrahlung
- 17d: Zustandsstrahlung nach Austritt aus dem Reflexionselement
- 17e: An der Eintrittsstelle reflektierte Zustandsstrahlung
- 17f: Divergente Zustandsstrahlung
- 17g: in die Testsubstanz transmittierte Zustandsstrahlung
- 17h: Divergente Zustandsstrahlung unter anderem Winkel als 17f
- 17i: durch Gasblasen, Partikel oder Oberflächendefekte an der Oberfläche gestreute Zustandsstrahlung
- 17k: Streustrahlung aus der Zustandsstrahlung
- 18: zweite Strahlungsquelle für die Zustandsstrahlung
- 19: zweite Strahlungsdetektor für gestreute Zustandsstrahlung
- 21, 23: dichroitischer Spiegel
- 22: Spiegel
- 24: der Reflexionsfläche gegenüberliegende Fläche des ATR-Kristalls 1
- 25: Auswertungseinrichtung
- 26: Photometer
- 27: Zustandssensor
- 28: Reflexionselement
- 100: Sensoranordnung

## Patentansprüche

1. Sensoranordnung (100) mit einem auf einer gestörten Totalreflexion beruhenden Photometer (26) zum Bestimmen einer optischen Eigenschaft einer Testsubstanz (2),
wobei das Photometer (26) umfasst
eine erste Strahlungsquelle (7), wobei die erste Strahlungsquelle (7) derart ausgestaltet ist, dass die erste Strahlungsquelle (7) in einem Betrieb der Sensoranordnung (100) elektromagnetische Messstrahlung erzeugt und abstrahlt,
ein Reflexionselement (28), wobei das Reflexionselement (28) eine mit der Testsubstanz (2) in Kontakt bringbare Reflexionsfläche (23) aufweist, wobei das Reflexionselement (28) derart in einem Strahlengang der von der ersten Strahlungsquelle (7) abgestrahlten Messstrahlung angeordnet und derart ausgestaltet ist, dass die Messstrahlung in dem Betrieb der Sensoranordnung (100) an der Reflexionsfläche (23) eine gestörte Totalreflexion erfährt,
einen ersten Strahlungsdetektor (9), wobei der erste Strahlungsdetektor (9) derart in dem Strahlengang der Messstrahlung angeordnet und derart ausgestaltet ist, dass der erste Strahlungsdetektor (9) in dem Betrieb der Sensoranordnung (100) die an der Reflexionsfläche (23) reflektierte Messstrahlung erfasst und ein die Leistung der erfassten Messstrahlung repräsentierendes Messsignal ausgibt, und
eine Auswertungseinrichtung (25), wobei die Auswertungseinrichtung (25) derart mit dem ersten Strahlungsdetektor (9) verbunden ist, dass die Auswertungseinrichtung (25) in dem Betrieb der Sensoranordnung (100) das Messsignal empfängt, und wobei die Auswertungseinrichtung (25) derart eingerichtet ist, dass die Auswertungseinrichtung (25) in dem Betrieb der Sensoranordnung (100) aus dem Messsignal die optische Eigenschaft der Testsubstanz bestimmt und ein Maß für die optische Eigenschaft ausgibt,
**dadurch gekennzeichnet, dass** die Sensoranordnung (100) einen Zustandssensor (27) aufweist,
wobei der Zustandssensor (27) ein Detektorelement (8, 9, 9a, 16, 19) umfasst, wobei das Detektorelement (8, 9, 9a, 16, 19) derart angeordnet und ausgestaltet ist, dass das Detektorelement (8, 9, 9a, 16, 19) in dem Betrieb der Sensoranordnung (100) ein Maß für die Leistung einer zumindest an der Reflexionsfläche (23) gestreuten oder durch die Reflexionsfläche (23) in die Testsubstanz transmittierten und in der Testsubstanz gestreuten elektromagnetischen Streustrahlung erfasst und ein das Maß für die Leistung der Streustrahlung repräsentierendes Zustandssignal ausgibt,
wobei die Auswertungseinrichtung (25) derart mit dem Detektorelement (8, 9, 9a, 16, 19) verbunden ist, dass die Auswertungseinrichtung (25) in dem Betrieb der Sensoranordnung (100) das Zustandssignal empfängt, und
wobei die Auswertungseinrichtung (25) derart eingerichtet ist, dass die Auswertungseinrichtung (25) in dem Betrieb der Sensoranordnung (100) aus dem Zustandssignal einen Status zumindest der Reflexionsfläche (23) oder der Testsubstanz (2) bestimmt.

2. Sensoranordnung (100) nach dem vorhergehenden Anspruch, wobei das Detektorelement ein zweiter Strahlungsdetektor ist (8, 9a, 16, 19), wobei der zweite Strahlungsdetektor (8, 9a, 16, 19) derart angeordnet und ausgestaltet ist, dass der zweite Strahlungsdetektor (8, 9a, 16, 19) in dem Betrieb der Sensoranordnung (100) die Streustrahlung (3k, 17m) erfasst und das die Leistung der erfassten Streustrahlung (3k, 17m) repräsentierende Zustandssignal ausgibt.

3. Sensoranordnung (100) nach Anspruch 1, wobei das Detektorelement der erste Strahlungsdetektor (9) oder ein zweiter Strahlungsdetektor (8, 9a, 16, 19) ist, wobei das Detektorelement (8, 9, 9a, 16, 19) derart angeordnet und ausgestaltet ist, dass das Detektorelement (8, 9, 9a, 16, 19) in dem Betrieb der Sensoranordnung (100) die an der Reflexionsfläche (23) reflektierte Messstrahlung (3b) oder eine an der Reflexionsfläche (23) reflektierte Zustandsstrahlung (17b) erfasst und das das Maß für die Leistung der Streustrahlung repräsentierende Zustandssignal ausgibt.

4. Sensoranordnung (100) nach einem der vorhergehenden Ansprüche, wobei die Streustrahlung (3k, 17m) die bei der gestörten Totalreflexion an der Reflexionsfläche (23) gestreute Messstrahlung oder ein an der Reflexionsfläche (23) oder in der Testsubstanz (2) gestreuter Anteil eines bei der gestörten Totalreflexion an der Reflexionsfläche (23) entstehenden evaneszenten Felds der Messstrahlung oder ein gestreuter Anteil eines Teils der durch die Reflexionsfläche (23) in die Testsubstanz (2) transmittierten Messstrahlung ist.

5. Sensoranordnung (100) nach dem vorhergehenden Anspruch, wobei die erste Strahlungsquelle (7) derart eingerichtet ist, dass die erste Strahlungsquelle (7) in dem Betrieb der Sensoranordnung (100) die Messstrahlung mit einer spektralen Bandbreite erzeugt und abstrahlt, wobei der erste Strahlungsdetektor (9) derart ausgestaltet ist, dass der erste Strahlungsdetektor (9) einen ersten Wellenlängenbereich aus der spektralen Bandbreite erfasst, wobei das Detektorelement (8, 9, 9a, 16, 19)derart ausgestaltet ist, dass das Detektorelement (8, 9, 9a, 16, 19)einen von dem ersten Wellenlängenbereich verschiedenen zweiten Wellenlängenbereich aus der spektralen Bandbreite erfasst, und wobei vorzugsweise der zweite Wellenlängenbereich kleinere Wellenlängen umfasst als der erste Wellenlängenbereich.

6. Sensoranordnung (100) nach einem der Ansprüche 1 bis 3, wobei der Zustandssensor (27) eine zweite Strahlungsquelle (18) umfasst, wobei die zweite Strahlungsquelle (18) derart ausgestaltet ist, dass die zweite Strahlungsquelle (18) in einem Betrieb der Sensoranordnung (100) elektromagnetische Zustandsstrahlung erzeugt und abstrahlt, und wobei das Reflexionselement (28) derart in einem Strahlengang der von der zweiten Strahlungsquelle (18) abgestrahlten Zustandsstrahlung angeordnet und derart ausgestaltet ist, dass die Zustandsstrahlung in dem Betrieb der Sensoranordnung (100) an der Reflexionsfläche (23) eine gestörte Totalreflexion erfährt oder durch die Reflexionsfläche (23) in die Testsubstanz transmittiert wird, so dass die von dem Detektorelement (8, 9, 9a, 16, 19) erfasste Streustrahlung ein bei der gestörten Totalreflexion an der Reflexionsfläche (23) gestreuter Anteil der Zustandsstrahlung oder ein an der Reflexionsfläche (23) oder in der Testsubstanz gestreuter Anteil des bei einer gestörten Totalreflexion an der Reflexionsfläche (23) entstehenden evaneszenten Felds der Zustandsstrahlung oder ein gestreuter Anteil der durch die Reflexionsfläche (23) in das Testmedium transmittierten Zustandsstrahlung ist.

7. Sensoranordnung (100) nach dem vorhergehenden Anspruch, wobei die zweite Strahlungsquelle (18) derart ausgestaltet ist, dass die zweite Strahlungsquelle (18) in einem Betrieb der Sensoranordnung (100) die Zustandsstrahlung mit einer Zustandswellenlänge erzeugt und abstrahlt, wobei die Zustandswellenlänge von einer Messwellenlänge der Messstrahlung verschieden ist und wobei die Zustandswellenlänge vorzugsweise kleiner ist als die Messwellenlänge.

8. Sensoranordnung (100) nach einem der Ansprüche 5 bis 7 soweit von Anspruch 1 abhängig, wobei der Zustandssensor (27) einen Referenzdetektor (9a) umfasst, wobei der Referenzdetektor (9a, 16) in einem Strahlengang eines Anteils der an der Reflexionsfläche (23) reflektierten Messstrahlung mit dem zweiten Wellenlängenbereich oder der an der Reflexionsfläche (23) reflektierten Zustandsstrahlung angeordnet und derart eingerichtet ist, dass der Referenzdetektor (9a, 16) in dem Betrieb der Sensoranordnung (100) eine Leistung des Anteils der Messstrahlung mit dem zweiten Wellenlängenbereich oder der Zustandsstrahlung erfasst und ein die Leistung der Messstrahlung mit dem zweiten Wellenlängenbereich oder der Zustandsstrahlung repräsentierendes Referenzsignal ausgibt, wobei die Auswertungseinrichtung (25) derart mit dem Referenzdetektor (9a, 16) verbunden ist, dass sie in dem Betrieb der Sensoranordnung (100) das Referenzsignal von dem Referenzdetektor (9a, 16) erhält, wobei die Auswertungseinrichtung (25) derart eingerichtet ist, dass sie in dem Betrieb der Sensoranordnung (100) das Zustandssignal mit dem Referenzsignal normiert.

9. Sensoranordnung (100) nach einem der vorhergehenden Ansprüche, wobei der zweite Strahlungsdetektor (8, 19) eine höhere Zeitauflösung aufweist als der erste Strahlungsdetektor (9).

10. Sensoranordnung (100) nach einem der vorhergehenden Ansprüche, wobei die Auswertungseinrichtung (25) derart eingerichtet ist, dass die Auswertungseinrichtung (25) in dem Betrieb der Sensoranordnung (100) das Messsignal und das Zustandssignal zeitlich korreliert und aus der zeitlichen Korrelation zwischen dem Messsignal und dem Zustandssignal eine Information zumindest über den Zustand der Reflexionsfläche (23) und/oder der Testsubstanz (2) oder über eine Vertrauenswürdigkeit des Messsignals ableitet.

11. Sensoranordnung (100) nach einem der vorhergehenden Ansprüche, wobei vor dem zweiten Strahlungsdetektor (8, 19) eine Blende (15) angeordnet ist, die ein Sichtfeld des zweiten Strahlungsdetektors (8, 19) begrenzt.

12. Sensoranordnung (100) nach einem der vorhergehenden Ansprüche, wobei vor dem zweiten Strahlungsdetektor (8, 19) eine Linse (13) angeordnet ist, wobei die Linse (13) derart ausgestaltet ist, dass sie ein Sichtfeld des zweiten Strahlungsdetektors (8, 19) auf die Reflexionsfläche (23) oder in die Testsubstanz (2) fokussiert.

13. Sensoranordnung (100) nach einem der vorhergehenden Ansprüche, wobei der erste Strahlungsdetektor (9) einen Spektrographen umfasst.

14. Verfahren zum Bestimmen einer optischen Eigenschaft einer Testsubstanz (2) mit den Schritten
in Kontakt Bringen einer Reflexionsfläche (23) eines Reflexionselements (28) mit der Testsubstanz (2),
Erzeugen und Abstrahlen von elektromagnetischer Messstrahlung,
Einstrahlen der Messstrahlung in das Reflexionselement (28), so dass die Messstrahlung an der Reflexionsfläche (23) eine gestörte Totalreflexion erfährt, Erfassen der an der Reflexionsfläche (23) reflektierten Messstrahlung,
Erzeugen eines die Leistung der erfassten Messstrahlung repräsentierenden Messsignals,
Bestimmen der optischen Eigenschaft der Testsubstanz aus dem Messsignal und
Ausgeben eines Maßes für die optische Eigenschaft,
**dadurch gekennzeichnet, dass** Verfahren weiterhin die Schritte aufweist
Erfassen eines Maßes für die Leistung von zumindest an der Reflexionsfläche (23) oder in der Testsubstanz gestreuter elektromagnetischer Streustrahlung, Erzeugen eines das Maß der Leistung der erfassten Streustrahlung repräsentierenden Zustandssignals und
Bestimmen Status zumindest der Reflexionsfläche (23) oder der Testsubstanz (2) aus dem Zustandssignal.

15. Verfahren nach Anspruch 14, wobei der Status zumindest der Reflexionsfläche (23) oder der Testsubstanz (2) aus dem momentanen Zustandssignal, aus einem zeitlichen Verlauf des Zustandssignals oder aus einer zeitlichen Korrelation zwischen dem zeitlichen Verlauf des Zustandssignals und einem zeitlichen Verlauf des Messsignals bestimmt wird.
